# EUROPEAN PATENT APPLICATION

(11) **EP 1 357 134 A2**
(43) Date of publication of application: **29.10.2003**
(21) Application number: 03011959.8
(22) Date of filing: 24.02.1998
(51) Int. Cl.: C08F 10/00, C08F 4/70, C07F 9/50, C07F 15/04, B01J 31/24, B01J 31/18

(54) **Catalyst compositions for the polymerization of olefins**

(30) Priority: 13.03.1997 US 40754 P; 18.04.1997 US 44691 P; 01.05.1997 US 45337 P; 02.05.1997 US 45357 P; 02.05.1997 US 45358 P; 06.05.1997 US 45697 P
(62) Divisional of application: 98906683.2
(71) Applicant: EASTMAN CHEMICAL COMPANY, Kingsport, TN 37660 (US)
(72) Inventor: Killian, Christopher Moore, Gray, Tennessee 37615 (US); McDevitt, Jason Patrick, Wake Forest, North Carolina 27587 (US); Mackenzie, Peter Borden, Kingsport, Tennessee 37660 (US); Moody, Leslie Shane, Johnson City, Tennessee 37604 (US); Ponasik, James Allen, Jr., 1410 Kingsport, Tennessee 37660 (US)
(74) Representative: Brown, Fraser Gregory James

(57) **Abstract**

The present invention includes novel ligands which may be utilized as part of a catalyst system. A catalyst system of the present invention is a transition metal - ligand complex. In particular, the catalyst system includes a transition metal component and a ligand component comprising a Nitrogen atom and/or functional groups comprising a Nitrogen atom, generally in the form of an imine functional group. In certain embodiments, the ligand component may further comprise a phosphorous atom. Preferred ligand components are bidentate (bind to the transition metal at two or more sites) and include a nitrogen - transition metal bond. The transition metal - ligand complex is generally cationic and associated with a weakly coordinating anion.

A catalyst system of the present invention may further comprise a Lewis or Bronsted acid. The Lewis or Bronsted acid may be complexed with the ligand component of the transition metal-ligand complex.

## Description

### Cross Reference to Related Applications

This application claims the benefit of the following provisional applications under 35 USC § 119: Provisional Application Serial No. 60/040,754, filed March 13, 1997; Provisional Application Serial No. 60/044,691, filed April 18, 1997; Provisional Application Serial No. 60/045,337, filed May 1, 1997; Provisional Application Serial No. 60/045,358, filed May 2, 1997; Provisional Application Serial No. 60/045,357, tiled May 2, 1997; and Provisional Application Serial No. 60/045,697, filed May 6, 1997.

### Field of the Invention

The present invention relates to catalyst compositions for olefin polymerization and processes for preparing polyolefins utilizing the catalysts. More particularly, the present invention relates to catalyst compositions comprising ligand complexes comprising nitrogen or nitrogen and phosphorus, for example, phosphine groups, heterocycle groups or imine groups.

### Background of the Invention

Olefin polymers are used in a wide variety of products, from sheathing for wire and cable to film. Olefin polymers are used, for instance, in injection or compression molding applications, in extruded films or sheeting, as extrusion coatings on paper, for example photographic paper and digital recording paper, and the like. Improvements in catalysts have made it possible to better control polymerization processes, and, thus, influence the properties of the bulk material. Increasingly, efforts are being made to tune the physical properties of plastics for lightness, strength, resistance to corrosion, permeability, optical properties, and the like, for particular uses. Chain length, polymer branching and functionality have a significant impact on the physical properties of the polymer. Accordingly, novel catalysts are constantly sought in attempts to obtain a catalytic process for polymerizing olefins which permits more efficient and better controlled polymerization of olefins.

Conventional polyolefins are prepared by a variety of polymerization techniques, including liquid phase, heterogeneous gas phase, and slurry polymerizations. Certain transition metal catalysts, such as those based on titanium compounds (e.g. TiCl₃ or TiCl₄) in combination with organoaluminum cocatalysts, are used to make linear and linear low density polyethylenes as well as poly-α-olefins such as polypropylene. These so-called "Ziegler-Natta" catalysts are quite sensitive to oxygen and are ineffective for the copolymerization of nonpolar and polar monomers.

Recent advances in non-Ziegler-Natta olefin polymerization catalysis include the following:
L. K. Johnson *et al*., WO Patent Application 96/23010, discloses the polymerization of olefins using cationic nickel, palladium, iron, and cobalt complexes containing diimine and bisoxazoline ligands. This document also describes the polymerization of ethylene, acyclic olefins, and/or selected cyclic olefins and optionally selected unsaturated acids or esters such as acrylic acid or alkyl acrylates to provide olefin homopolymers or copolymers;
European Patent Application Serial No. 381,495, describes the polymerization of olefins using palladium and nickel catalysts which contain selected bidentate phosphorous containing ligands;
L. K. Johnson *et al., J. Am. Chem. Soc*., **1995**, *117*, 6414, describes the polymerization of olefins such as ethylene, propylene, and 1-hexene using cationic α-diimine-based nickel and palladium complexes. These catalysts have been described to polymerize ethylene to high molecular weight branched polyethylene. In addition, Pd complexes act as catalysts for the polymerization and copolymerization of olefins and methyl acrylate (L. K. Johnson *et al*., *J. Am. Chem. Soc*., **1996**, *118*, 267).
G.F. Schmidt *et al., J. Am. Chem.* Soc. **1985**, *107*, 1443, describes a cobalt(III) cyclopentadienyl catalytic system having the structure [C₅Me₅(L)CoCH₂CH₂-µ-H]⁺, which provides for the "living" polymerization of ethylene. In addition, M. Brookhart et al., *Macromolecules* **1995,** *28*, 5378, discloses using such "living" catalysts in the synthesis of end-functionalized polyethylene homopolymers;
U. Klabunde, U. S. Patent Nos. 4,906,754; 4,716,205; 5,030,606; 5,175,326; describes the conversion of ethylene to polyethylene using anionic phosphorous/oxygen donors ligated to Ni(II). The polymerization reactions were run between 25 and 100 °C with modest yields, preparing linear polyethylene having a weight-average molecular weight ranging between 8K and 350K. In addition, Klabunde describes the preparation of copolymers of ethylene and functional group containing monomers;
M. Peuckert *et al*., *Organomet.* **1983,** *2*(5), 594, discloses the oligomerization of ethylene using phosphine/carboxylate donors ligated to Ni(II), which demonstrated modest catalytic activity (0.14 to 1.83 TO/s). The oligomerizations were carried out at 60 to 95 °C and 10 to 80 bar ethylene in toluene, to produce linear α-olefins;
R.E. Murray, U.S. Patents Nos. 4,689,437 and 4,716,138, describes the oligomerization of ethylene using phosphine/sulfonate donors ligated to Ni(II). These complexes show catalyst activities approximately 15 times greater than those reported with phosphine/carboxylate analogs;
W. Keim *et al*., *Angew. Chem. Int. Ed. Eng.* **1981,** *20*, 116, and V.M. Mohring, *et al., Angew. Chem. Int. Ed. Eng.* **1985,** 24,1001, disclose the polymerization of ethylene and the oligomerization of α-olefins with aminobis(imino)phosphorane nickel catalysts; G. Wilke, *Angew. Chem. Int.* *Ed. Engl.* **1988,** *27*, 185, describes a nickel allyl phosphine complex for the polymerization of ethylene.
K.A.O. Starzewski *et al., Angew. Chem. Int. Ed. Engl.* **1987,** *26*, 63 and U. S. Patent 4,691,036, describes a series of bis(ylide)nickel complexes, used to polymerize ethylene to provide high molecular weight linear polyethylene;
WO Patent Application 97/02298, discloses the polymerization of olefins using a variety of neutral N, O, P, or S donor ligands, in combination with a nickel(0) compound and an acid;
Brown *et al*., WO 97/17380, describes the use of Pd α-diimine catalysts for the polymerization of olefins including ethylene in the presence of air and moisture;
Fink *et al*., U. S. Patent No. 4,724,273, describes the polymerization of α-olefins using aminobis(imino)phosphorane nickel catalysts and the compositions of the resulting poly(α-olefins);
Additional recent developments are described by Sugimura *et al*., in JP96-84344, JP96-84343, and WO 9738024, and by Yorisue *et al*., in JP96-70332

Notwithstanding these advances in non-Ziegler-Natta catalysis, there remains a need for efficient and effective Group 8-10 transition metal catalysts for effecting polymerization of olefins. In addition, there is a need for novel methods of polymerizing olefins employing such effective Group 8-10 transition metal catalysts. In particular, there remains a need for Group 8-10 transition metal olefin polymerization catalysts with both improved temperature stability and functional group compatibility. Further, there remains a need for a method of polymerizing olefins utilizing effective Group 8-10 transition metal catalysts in combination with a Lewis acid so as to obtain a catalyst that is more active and more selective.

### Summary of the Invention

The present invention includes novel ligands, which may be utilized as part of a catalyst system. A catalyst system of the present invention is a transition metal - ligand complex. In particular, the catalyst system is comprised of a transition metal component and a ligand component comprising a nitrogen atom, generally in the form of an imine or heterocycle group. In certain embodiments, the ligand component may further comprise a phosphorous atom. Preferred ligand components are bidentate and include a nitrogen - transition metal bond. The transition metal - ligand complex is generally cationic and associated with a weakly coordinating anion.

A catalyst system of the present invention may further comprise a Lewis or Bronsted acid. The Lewis or Bronsted acid may be complexed with the transition metal component and/or the ligand component of the transition metal-ligand complex.

In one aspect of the present invention, the transition metal component of the catalyst system is Group 8-10 transition metals. In another aspect, the catalyst system further comprises a Lewis or Bronsted acid and the transition metal component is Group 8-10 transition metals. Preferred transition metal components include iron (Fe), cobalt (Co), nickel (Ni) and palladium (Pd). The choice of a particular transition metal may be made in view of the end use of the catalyst system.

The present invention also provides processes for preparing polyolefins, preferably having a degree of polymerization of at least 10, comprising contacting one or more monomers, preferably selected from the group comprising R'CH=CHR" wherein R' and R" are independently hydrogen, hydrocarbyl, fluoralkyl, or R' and R" collectively form a cyclic olefin, with a catalyst system of the present invention at a temperature and a pressure sufficient to effect polymerization, preferably a temperature of -100 to 200 °C, and a pressure of from about 1 atmosphere to 100 atmospheres.

The catalyst system of this invention is extremely versatile in that changes in the ligand or changes to the transition metal itself can be made to obtain a "tailor made" catalyst to suit a particular set of requirements for a particular monomer and polymer. In certain embodiments of the present invention, a Lewis or Bronsted acid may be used as a co-catalyst to render the transition metal more electron deficient, and therefore more active and/or selective, and/or act as a site to bind the catalyst to a surface.

Further features and advantages of the catalyst system and processes of the present invention will become more apparent from the following more detailed description.

### Detailed Description of the Invention

The novel ligand components of the present invention are broadly described as comprising a nitrogen atom, preferably in the form of an imine or heterocyclic functional group.

A catalyst system of the present invention may further comprise a Lewis or Bronsted acid, which may be complexed with the ligand component or the transition metal component. While not wishing to be bound by theory, it is believed that the Lewis acid complexation can render the transition metal more electron deficient, and therefore more active and/or selective, and/or act as a site to bind the catalyst to a surface. Although this strategy may be implemented in a variety of ways, ligands in which the Lewis acid is bound to one or more heteroatoms which are π-conjugated to the donor atom or atoms bound to the transition metal are preferred.

In one aspect of the present invention, the transition metal component of the catalyst system is a Group 8-10 transition metal. In another aspect, the catalyst system further is a Lewis or Bronsted acid and the transition metal component is a Group 8-10 transition metal. A catalyst system of the present invention may advantageously be utilized in a process for the polymerization of olefins, including ethylene and α-olefins such as propylene and 1-hexene and cyclic olefins such as cyclopentene and norbornene. Accordingly, a process of the present invention is contacting one or more monomers comprising R'CH=CHR" with a catalyst system of the present invention at a temperature and a pressure sufficient to effect polymerization, preferably a temperature of -100 to 200 °C, more preferably a temperature from 25 to 150 °C, and a pressure of from about 1 atmosphere to 100 atmospheres, wherein R' and R" are independently hydrogen, hydrocarbyl, fluoroalkyl, or R' and R" may be linked to form a cyclic olefin. The possible embodiments of a process of the present invention for the production of polyolefins include processes utilizing the catalyst systems of the present invention described herein.

The embodiments of a catalyst system of the present invention are described in detail below utilizing the following terms defined as follows:
Symbols ordinarily used to denote elements in the Periodic Table take their ordinary meaning, unless otherwise specified. Thus, N, O, S, P, and Si stand for nitrogen, oxygen, sulfur, phosphorus and silicon, respectively.
Examples of neutral Lewis bases include, but are not limited to, ethers, organic nitriles or organic sulfides.

Examples of Lewis acids include, but are not limited to, methylalumoxane (hereinafter MAO) and other aluminum sesquioxides, R⁷₃Al, R⁷₂AlCl, R⁷AlCl₂ (where R⁷ is alkyl), organoboron compounds, boron halides, B(C₆F₅)₃, R⁹₃Sn[BF₄], (where R⁹ is alkyl or aryl), MgCl₂, and H⁺X⁻, where X⁻ is a weakly coordinating anion.

A "hydrocarbyl" group means a monovalent or divalent, linear, branched or cyclic group which contains only carbon and hydrogen atoms. Examples of monovalent hydrocarbyls include the following: C₁-C₂₀ alkyl; C₁-C₂₀ alkyl substituted with one or more groups selected from C₁-C₂₀ alkyl, C₃-C₈ cycloalkyl or aryl; C₃-C₈ cycloalkyl; C₃-C₈ cycloalkyl substituted with one or more groups selected from C₁-C₂₀ alkyl, C₃-C₈ cycloalkyl or aryl; C₆-C₁₄ aryl; and C₆-C₁₄ aryl substituted with one or more groups selected from C₁-C₂₀ alkyl, C₃-C₈ cycloalkyl or aryl. As used herein, the term "aryl" preferably denotes a phenyl, napthyl, or anthracenyl group. When the above groups are substituted, they are preferably substituted from one to four times with the listed groups. Examples of divalent (bridging hydrocarbyls) include: -CH₂-, -CH₂CH₂-, -C₆H₄-, and -CH₂CH₂CH₂-.

Specific examples of the group G as used herein include but are not limited to: -CH₂-CH₂-, -CH=CH-, -CH₂-. and -C₆H₄-. Specific examples of G¹ as used herein include, but are not limited to: -CH₂-CH₂-CH₂-, -CH₂-CH₂-CH₂-CH₂-, -O-CH₂-C(CH₃)₂-, -S-CH=CH-, -S-CH₂-CH₂-, -O-CH=CH-, -N=CH-CH=CH-, -CH=CH-CH=CH-, -CH=N-CH=CH-, -NPh-CH=CH-, and -N(CH₃)-C₆H₄-,. Specific examples of G² as used herein include, but are not limited to: -CH₂-CH₂-, -CH₂-O-, -N(CH₃)-CH₂-, -CH₂-CH₂-O-, -N(CH₃)-CH₂-CH₂-, -S-CH₂-, -S-CH₂-CH₂-, -CH=CH-, -CH=N-, -CH=CH-CH₂-, -CH=N-CH₂-, and -C₆H₄-.

A "silyl" group refers to a SiR₃ group where Si is silicon and R is hydrocarbyl or substituted hydrocarbyl or silyl, as in Si(SiR₃)_{3.}

A "heteroatom" refers to an atom other than carbon or hydrogen. Preferred heteroatoms include oxygen, nitrogen, phosphorus, sulfur, selenium, arsenic, chlorine, bromine, and fluorine.

The term "fluoroalkyl" as used herein refers to a C₁-C₂₀ alkyl group substituted with one or more fluorine atoms.

A "substituted hydrocarbyl" refers to a monovalent or divalent hydrocarbyl substituted with one or more heteroatoms. Examples of monovalent substituted hydrocarbyls include: trifluoromethyl, 2,6-dimethyl-4-methoxyphenyl, 2,6-diisopropyl-4-methoxyphenyl, 4-cyano-2,6-dimethylphenyl, 2,6-dimethyl-4-nitrophenyl, 2,6-difluorophenyl, 2,6-dibromophenyl, 2,6-dichlorophenyl, 4-methoxycarbonyl-2,6-dimethylphenyl, 2-tert-butyl-6-chlorophenyl, 2,6-dimethyl-4-phenylsulfonylphenyl, 2,6-dimethyl-4-trifluoromethylphenyl, 2,6-dimethyl-4-trimethylammoniumphenyl (associated with a weakly coordinating anion), 2,6-dimethyl-4-hydroxyphenyl, 9-hydroxyanthr-10-yl, 2-chloronapth-1-yl, 4-methoxyphenyl, 4-nitrophenyl, and 9-nitroanthr-10-yl. Examples of divalent substituted hydrocarbyl include: 4-methoxy-1,2-phenylene, 1-methoxymethyl-1,2-ethanediyl, 1,2-bis(benzyloxymethyl)-1,2-ethanediyl, and 1-(4-methoxyphenyl)-1,2-ethanediyl.

A "mono-olefin" means a hydrocarbyl group having one carbon-carbon double bond.

A "suitable nickel precursor" refers to a nickel compound which may be combined with compound **III, VI, IX,** or **XII,** and optionally a Lewis or Bronsted acid, to form an active olefin polymerization catalyst. Examples include: bis(1,5-cycloctadiene)nickel(0), (1,2-dimethoxyethane)nickel(II) dibromide, bis[(chloro)(1, 2, 3-η³-2-propenyl) nickel(II)].

The term "polymer" as used herein is meant a species comprised of monomer units and having a degree of polymerization (DP) of ten or higher.

The term "weakly coordinating counterion" is well-known in the art *per se* and generally refers to a large bulky anion capable of delocalization of the negative charge of the anion. Suitable weakly coordinating anions include, but are not limited to, PF₆⁻, BF₄⁻, SbF₆⁻, (Ph)₄B⁻ where Ph = phenyl, ⁻BAr₄ where ⁻BAr₄ = tetrakis[3,5-bis(trifluoromethyl)phenyl]borate. The coordinating ability of such anions is known and described in the literature (Strauss, S. *et al*., *Chem. Rev.* **1993,** *93*, 927).

As used herein, the terms "monomer" or "olefin monomer" refer to the olefin or other monomer compound before it has been polymerized. The term "monomer units" refers to the moieties of a polymer that correspond to the monomers after they have been polymerized;

In some cases, a compound Y is required as a cocatalyst. Suitable compounds Y include a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion. Preferred compounds Y include: methylalumoxane (hereinafter MAO) and other aluminum sesquioxides, R⁷₃Al, R⁷₂AlCl, R⁷AlCl₂ (where R⁷ is alkyl), organoboron compounds, boron halides, B(C₆F₅)₃, R⁹₃Sn[BF₄], (where R⁹ is alkyl or aryl), MgCl₂, and H⁺X⁻, where X⁻ is a weakly coordinating anion.

Especially preferred olefin monomers include ethylene, propylene, 1-butene, 1-pentene, 1-hexene, 1-heptene, cyclopentene, and norbornene.

### First Embodiment of the Present Invention

In a first embodiment, the present invention provides a catalyst system comprising: an imine/phosphine ligand. In a first aspect, the catalyst system is a compound of the formula **I:** 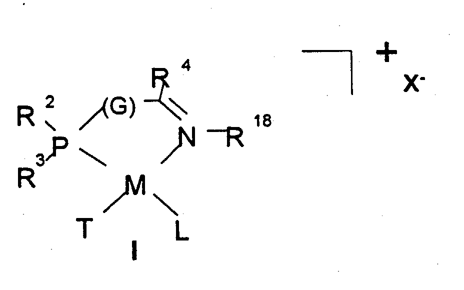 wherein:
R² and R³ are each independently selected from hydrocarbyl, or substituted hydrocarbyl, and in addition, R² and R³ may be taken together with phosphorus to form a ring;
G is hydrocarbyl or substituted hydrocarbyl;
R⁴ is hydrogen, hydrocarbyl, substituted hydrocarbyl, or silyl;
R¹⁸ is hydrocarbyl or substituted hydrocarbyl;
T is hydrogen or hydrocarbyl group;
L is a monoolefin or a neutral Lewis base where the donating atom is nitrogen, oxygen, or sulfur;
M is Ni(II), Pd(II), or Co(II); and
X⁻ is a weakly coordinating anion.

In a second aspect, this first embodiment of the present invention provides a catalyst system formed by contacting a first compound Y with a second compound **II** having the formula: 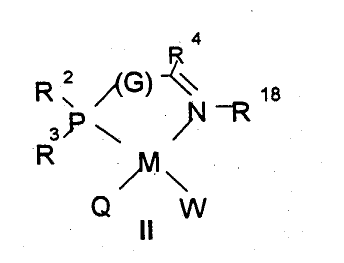 wherein:
R² and R³ are each independently selected from hydrocarbyl, or substituted hydrocarbyl, and in addition, R² and R³ may be taken together with phosphorus to form a ring;
G is hydrocarbyl or substituted hydrocarbyl and may comprise a cyclic olefin;
R⁴ is hydrogen, hydrocarbyl, substituted hydrocarbyl, or silyl;
R¹⁸ is hydrocarbyl or substituted hydrocarbyl;
Q is hydrocarbyl, chloride, iodide or bromide;
W is hydrocarbyl, chloride, iodide or bromide;
M is Ni(II), Pd(II), or Co(II);
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

Preferred neutral Lewis acids include: MAO and other aluminum sesquioxides, R₃⁷Al, R⁷₂AlCl, R⁷AlCl₂ where R⁷ is alkyl. Complex **II** and compound Y may be combined in the liquid phase. The liquid phase may include solvent or neat monomer. In a process for preparing polyolefins, complex **II** and compound Y may be combined in the presence of monomer. The molar ratio of compound Y to complex **II** may be from about 10 to 10,000.

In a third aspect, the first embodiment of the present invention provides a compound **III** of the formula: 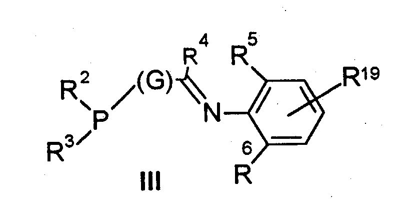 wherein
R² and R³ are each independently selected from hydrocarbyl, or substituted hydrocarbyl, and in addition, R² and R³ may be taken together with phosphorus to form a ring;
G is hydrocarbyl or substituted hydrocarbyl;
R¹⁹ is hydrogen, hydrocarbyl, substituted hydrocarbyl, heteroatom connected hydrocarbyl, or nitro;
R⁴ is hydrogen, hydrocarbyl, substituted hydrocarbyl, or silyl;
R⁵ and R⁶ are each independently selected from hydrogen, hydrocarbyl, substituted hydrocarbyl or a halide; provided that R⁵ and R⁶ are not both hydrogen.

The present invention also provides a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst comprising a compound of formula **I:** 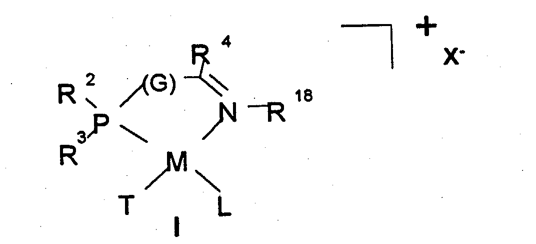 wherein:
R² and R³ are each independently selected from hydrocarbyl, or substituted hydrocarbyl, and in addition, R² and R³ may be taken together with phosphorus to form a ring;
G is hydrocarbyl or substituted hydrocarbyl;
R⁴ is hydrogen, hydrocarbyl, substituted hydrocarbyl, or silyl;
R¹⁸ is hydrocarbyl or substituted hydrocarbyl;
T is hydrogen or hydrocarbyl;
L is a monoolefin or a neutral Lewis base where the donating atom is nitrogen, oxygen, or sulfur;
M is Ni(II), Pd(II), or Co(II); and
X⁻ is a weakly coordinating anion; at a temperature and a pressure sufficient to effect polymerization.

In a further preferred embodiment, there is provided a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst system wherein the catalyst is formed by contacting a first compound Y with a second compound **II** having the formula: 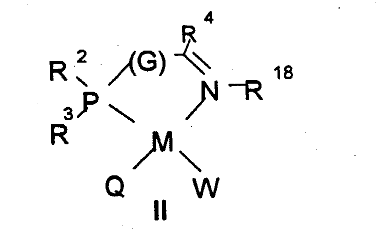 wherein:
R² and R³ are each independently selected from hydrocarbyl, or substituted hydrocarbyl, and in addition, R² and R³ may be taken together with phosphorus to form a ring;
G is hydrocarbyl or substituted hydrocarbyl;
R⁴ is hydrogen, hydrocarbyl, substituted hydrocarbyl, or silyl;
R¹⁸ is hydrocarbyl or substituted hydrocarbyl;
Q is hydrocarbyl, chloride, iodide or bromide;
W is hydrocarbyl, chloride, iodide or bromide;
M is Ni(II), Pd(II), or Co(II);
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion at a temperature and a pressure sufficient to effect polymerization.

In a further preferred embodiment, there is provided a process for the polymerization of olefins comprising contacting a compound of the formula III with a suitable nickel precursor and one or more olefin monomers of the formula R'CH=CHR" wherein R' and R" are independently hydrogen, hydrocarbyl, fluoroalkyl, or R' and R" may be linked to form a cyclic olefin: 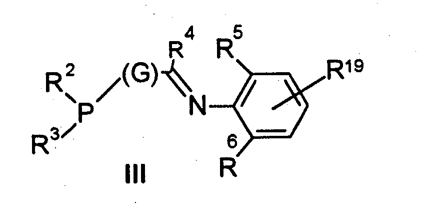 wherein
R² and R³ are each independently selected from hydrocarbyl or substituted hydrocarbyl, or R² and R³ may be taken together with phosphorus to form a ring;
G is hydrocarbyl or substituted hydrocarbyl and may comprise a cyclic olefin;
R¹⁹ is hydrogen, hydrocarbyl, substituted hydrocarbyl, heteroatom connected hydrocarbyl, or nitro;
R⁴ is hydrogen, hydrocarbyl, substituted hydrocarbyl, or silyl; and
R⁵ and R⁶ are each independently selected from hydrogen, hydrocarbyl, substituted hydrocarbyl or halide; provided that R⁵ and R⁶ are not both hydrogen.

Compound **III** may be utilized as a precursor to complex **I** or **II.**

Complexes **I** and **II,** may be synthesized by a method analogous to that described in the literature (Johnson *et al., J. Am. Chem. Soc.* **1995,** *117*, 6414).

The skilled artisan, in possession of this disclosure, could make the present compounds without undue experimentation. Methods of synthesizing complexes **I, II,** and compound **III,** are also illustrated in the following examples.

Preferred ligand components for use in complex **I** or **II** and preferred catalyst systems utilizing complex **I** or **II** are also set forth in the following examples.

Preferred polyolefin products will have a degree of polymerization of at least 10. A preferred olefin monomer is R'CH=CHR" wherein R' and R" are independently hydrogen, hydrocarbyl, fluoroalkyl, or R' and R" may be linked to form a cyclic olefin. The preferred temperature range of about -100 to 200 °C, more preferably a temperature from about 25 to 150 °C; a preferred pressure is about 1 atmosphere to 100 atmospheres.

Examples of processes for preparing polyolefins utilizing complex **I** or complex **II** of the first embodiment of the present invention are set forth in the following examples.

### Second-Fourth Embodiments of the Present Invention

The second, third, and fourth embodiments of the present invention provide catalyst systems comprising transition metal-ligand complexes
wherein the ligand component is a bidentate ligand which binds to a transition metal and is a heterocycle and an additional nitrogen donor group such as an imine or imidate ester.

### Second Embodiment of the Present Invention

The second embodiment of the present invention provides a catalyst system comprising a transition metal complex of bidentate ligands (imino-substituted heterocycles) having a five-membered ring, formed by the metal complex, and comprising one metal atom, one carbon atom, and three nitrogen atoms.

The olefin polymerization catalysts of the second embodiment of the present invention comprise transition metal complexes of bidentate ligands that can be referred to as imino-substituted heterocycles, more specifically imino-substituted pyrazoles and other related heterocycles with adjacent 1,2-nitrogen atoms. The transition metal component is a Group 8-10 transition metal. Nickel, cobalt and palladium are preferred transition metals. The ligands may also be referred to using a "imine/heterocycle" or "heterocycle/ imine" nomenclature which describes the two components of the bidentate ligand, e.g., imine/triazole. The five-membered ring formed by the transition metal complex contains one transition metal atom, one carbon atom, and three nitrogen atoms (two of which are provided by the heterocycle component of the bidentate ligand).

In a first aspect the second embodiment of the present invention provides a catalyst system comprising a transition metal-ligand complex of the formula **IV**: 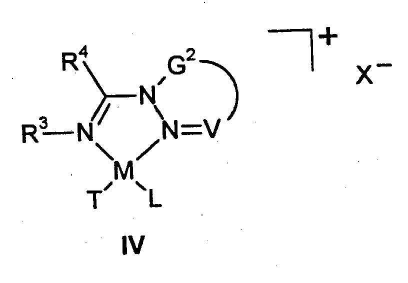 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
T is hydrogen, hydrocarbyl, or substituted hydrocarbyl;
L is a monoolefin or a neutral Lewis base where the donating atom is nitrogen, oxygen, sulfur;
M is Ni(II), Pd(II), or Co(II); and
X⁻ is a weakly coordinating anion.

A preferred embodiment of the present invention involves a catalyst of the formula **XVI**: 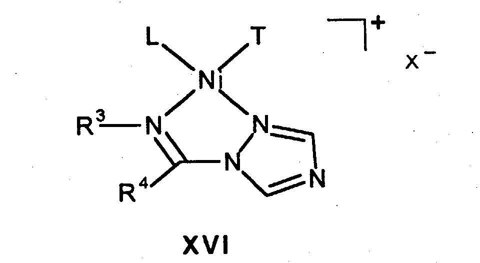 wherein:
R³ is hydrocarbyl or substituted hydrocarbyl;
R⁴ is hydrogen, hydrocarbyl, substituted hydrocarbyl, or silyl;
T is hydrogen, hydrocarbyl, or substituted hydrocarbyl;
L is a monoolefin or a neutral Lewis base where the donating atom is nitrogen, oxygen or sulfur; and
X⁻ is a weakly coordinating anion.

In a second aspect, this second embodiment of the present invention provides a catalyst system formed by contacting a first compound Y with a second compound **V** having the formula: 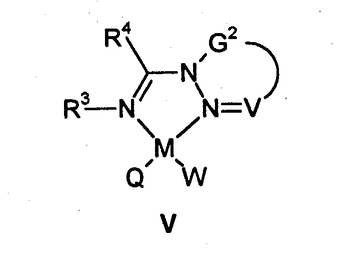 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
Q is hydrocarbyl, chloride, iodide, or bromide;
W is hydrocarbyl, chloride, iodide, or bromide;
M is Ni(II), Pd(II), or Co(II);
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

Preferred neutral Lewis acids include: MAO and other aluminum sesquioxides, R₃⁷Al, R⁷₂AlCl, and R⁷AlCl₂ wherein R⁷ is alkyl. Complex **V** and compound Y may be combined in the liquid phase. The liquid phase may include solvent or neat monomer. In a process for preparing polyolefins, complex **V** and compound **Y** may be combined in the presence of monomer. The molar ratio of compound **Y** to complex **V** may be from about 10 to 10,000.

A preferred embodiment of the present invention involves a catalyst of the formula XVII: 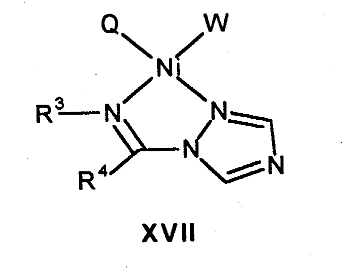 wherein:
R³ is hydrocarbyl or substituted hydrocarbyl;
R⁴ is hydrogen, hydrocarbyl, substituted hydrocarbyl, or silyl;
Q is a hydrocarbyl, chloride, iodide, or bromide; and
W is a hydrocarbyl, chloride, iodide, or bromide.

In a third aspect, the second embodiment of present invention provides a compound **VI** of the formula: 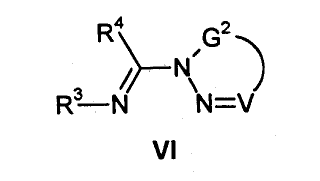 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;

Compound **VI** may be utilized as a precursor to complex **IV** or **V.**

Complexes **IV** and **V** may be synthesized by a method analogous to that described in the literature (Johnson *et al., J. Am. Chem. Soc.* **1995,** *117*, 6414).

The skilled artisan, in possession of this disclosure, could make the present compounds without undue experimentation. Methods of synthesizing complexes **IV** and **V,** and compound **VI** are also illustrated in the following examples.

Preferred ligand components for use in complex IV or V and preferred catalyst systems utilizing complex **IV** or **V** are also set forth in the following examples.

The present invention also provides processes for preparing polyolefins utilizing complexes **IV, V,** and compound **VI**.

Thus, the present invention provides a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with the a catalyst comprising a compound of formula **IV**: 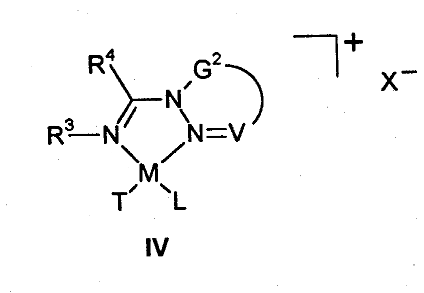 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
T is hydrogen, hydrocarbyl, or substituted hydrocarbyl;
L is a monoolefin or a neutral Lewis base where the donating atom is nitrogen, oxygen or sulfur;
M is Ni(II), Pd(II), or Co(II); and X⁻ is a weakly coordinating anion; at a temperature and a pressure sufficient to effect polymerization.

Accordingly, the invention also provides a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst wherein the catalyst is formed by contacting a first compound Y with a second compound V having the formula: 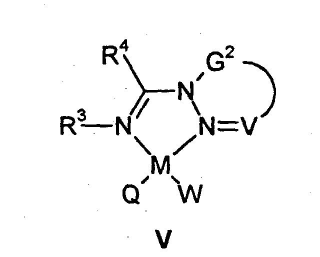 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
Q is hydrocarbyl, chloride, iodide, or bromide;
W is hydrocarbyl, chloride, iodide, or bromide;
M is Ni(II), Pd(II), or Co(II);
and Y is a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion; at a temperature and a pressure sufficient to effect polymerization.

The invention also provides a process for the polymerization of olefins comprising contacting a compound of the formula VI with a suitable nickel precursor, optionally a Bronsted or Lewis acid and one or more olefin monomers of the formula R'CH=CHR" wherein R' and R" are independently hydrogen, hydrocarbyl, fluoroalkyl, or R' and R" may be linked to form a cyclic olefin: 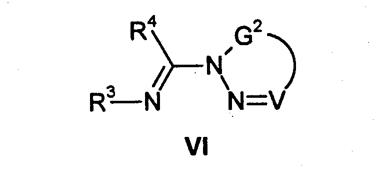 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus.

The invention also provides processes for preparing polyolefins utilizing the compounds of formula **XVI** and **XVII.**

Thus, the present invention provides a process for preparing polyolefins comprising contacting one or more olefin monomers having the composition R'CH=CHR" wherein R' and R" are independently hydrogen, hydrocarbyl, fluoroalkyl, or R' and R" may be linked to form a cyclic olefin; with a catalyst comprising the formula **XVI**: 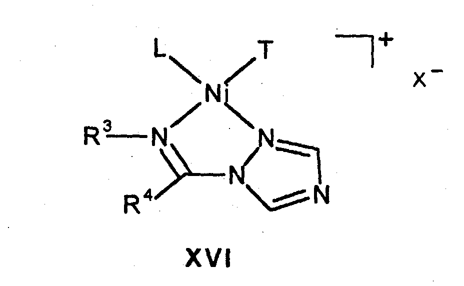 wherein:
R³ is hydrocarbyl or substituted hydrocarbyl;
R⁴ is hydrogen, hydrocarbyl, substituted hydrocarbyl, or silyl;
T is hydrogen, hydrocarbyl, or substituted hydrocarbyl;
L is a monoolefin or a neutral Lewis base where the donating atom is nitrogen, oxygen or sulfur; and
X⁻ is a weakly coordinating anion at a temperature and a pressure sufficient to effect polymerization.

Accordingly, the invention also provides a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with the catalyst system comprising a compound of the formula **XVII** and a second compound Y: 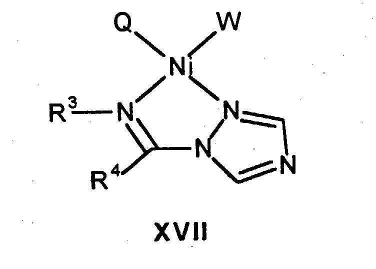 wherein:
R³ is hydrocarbyl or substituted hydrocarbyl;
R⁴ is hydrogen, hydrocarbyl, substituted hydrocarbyl, or silyl;
Q is hydrocarbyl, chloride, iodide, or bromide; and
W is hydrocarbyl, chloride, iodide, or bromide; and Y is a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion; at a temperature and a pressure sufficient to effect polymerization.

Preferred polyolefin products will have a degree of polymerization (DP) of at least 10. A preferred olefin monomer is R'CH=CHR" wherein R' and R" are independently hydrogen, hydrocarbyl, fluoroalkyl, or R' and R" may be linked to form a cyclic olefin. Preferred temperature conditions are -100 to 200 °C. Preferred pressure conditions are from about 1 atmosphere to 100 atmospheres.

Examples of processes for preparing polyolefins utilizing complex **IV** or **V** of the second embodiment of the present invention are set forth in the following examples.

### Third Embodiment of the Present Invention

The third embodiment of the present invention provides a class of olefin polymerization catalysts based on late transition metal complexes of bidentate ligands comprising one imidate ester, thioimidate ester, selenoimidate ester, or amidine N-donor fragment, and one heterocyclic N-donor fragment, the two fragments together comprising a neutral bidentate, N,N-donor ligand. In one aspect, the catalyst "system" further comprises a Lewis or Bronsted acid.

In a first aspect, the third embodiment of the present invention provides a catalyst system comprising a transition metal-ligand complex of the formula **VII**: 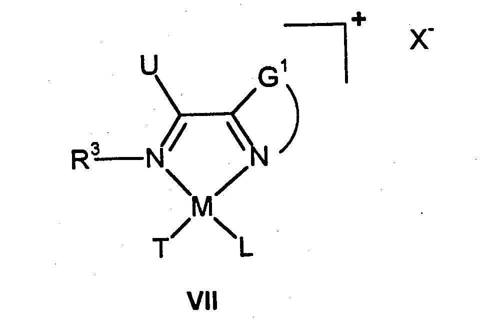 wherein:
R³ is hydrocarbyl, substituted hydrocarbyl or silyl;
U is is OR⁴, SR⁴, SeR4 or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N;
T is hydrogen, hydrocarbyl, or substituted hydrocarbyl;
L is a monoolefin or a neutral Lewis base where the donating atom is nitrogen, oxygen or sulfur;
M is Ni(II), Pd(II), or Co(II); and
X⁻ is a weakly coordinating anion.

In a second aspect this third embodiment of the present invention provides a catalyst system formed by contacting a first compound Y with a second compound **VIII** having the formula: 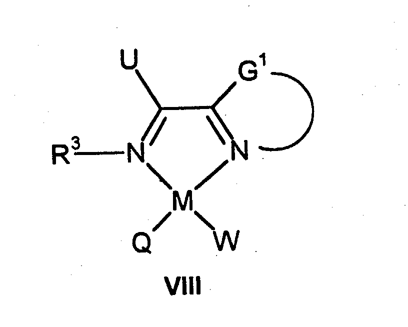 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N;
M is Ni(II), Pd(II), or Co(II);
Q is hydrocarbyl, chloride, iodide, or bromide;
W is hydrocarbyl, chloride, iodide, or bromide; and Y is selected from the group consisting of a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

Preferred neutral Lewis acids include: MAO and other aluminum sesquioxides, R₃⁷Al, R⁷₂AlCl, and R⁷AlCl₂ wherein R⁷ is alkyl. Complex **VIII** and compound Y may be combined in the liquid phase. The liquid phase may include solvent or neat monomer. In a process for preparing polyolefins, complex **VIII** and compound **Y** may be combined in the presence of monomer. The molar ratio of compound **Y** to complex **VIII** may be from about 10 to 10,000.

In a third aspect, the third embodiment of the present invention provides a compound **IX** of the formula: 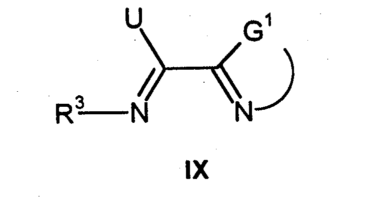 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen; and
G¹ is hydrocarbyl or substituted hydrocarbyl and may comprise a carbocyclic or heterocyclic ring, thereby forming a 5-membered or 6-membered heterocyclic ring comprising G¹, C, and N.

Compound **IX** may be utilized as a precursor to complex **VII** or **VIII.**

Complexes **VII** and **VIII** may be synthesized by a method analogous to that described in the literature (Johnson *et al., J. Am. Chem. Soc.* **1995,** *117*, 6414).

The skilled artisan, in possession of this disclosure, could make the present compounds without undue experimentation. Methods of synthesizing complexes **VII** and **VIII**, and compounds **IX**, are also illustrated in the following examples.

Preferred ligand components for use in complex **VII** or **VIII** and preferred catalyst systems utilizing complex **VII** or **VIII** are also set forth in the following examples.

The present invention also provides processes for preparing polyolefins utilizing complexes **VII**, **VIII**, and compound **IX**.

Thus, the present invention provides a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst comprising a compound of the formula **VII**: 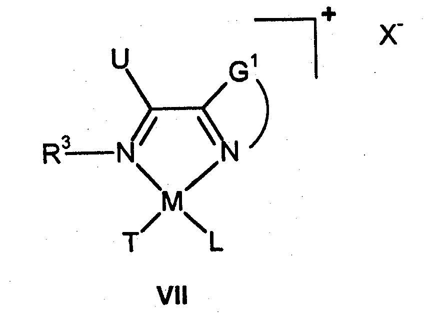 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N;
T is hydrogen, hydrocarbyl, or substituted hydrocarbyl;
L is a monoolefin or a neutral Lewis base where the donating atom is nitrogen, oxygen or sulfur;
M is Ni(II), Pd(II), or Co(II); and X⁻ is a weakly coordinating anion; at a temperature and a pressure sufficient to effect polymerization.

The invention also provides a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst system wherein the catalyst is formed by contacting a first compound Y with a second compound **VIII** having the formula: 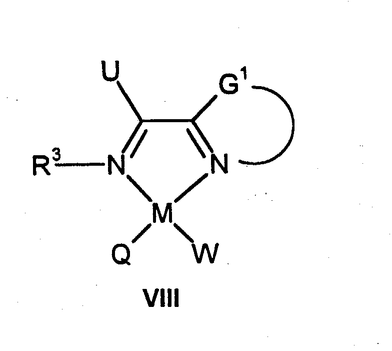 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N;
M is Ni(II), Pd(II), or Co(II);
Q is an hydrocarbyl, chloride, iodide, or bromide;
W is hydrocarbyl, chloride, iodide, or bromide;
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion; at a temperature and a pressure sufficient to effect polymerization.

The invention also provides a process for the polymerization of olefins comprising contacting a compound of the formula IX with a suitable nickel precursor, optionally a Lewis or Bronsted acid and one or more olefin monomers of the formula R'CH=CHR" wherein R' and R" are independently hydrogen, hydrocarbyl, fluoroalkyl, or R' and R" may be linked to form a cyclic olefin: 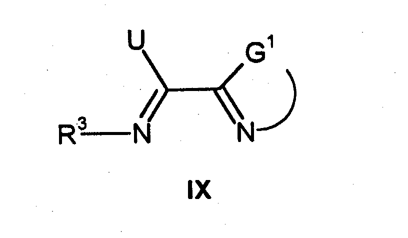 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N.

Preferred polyolefin products will have a degree of polymerization (DP) of at least 10. A preferred olefin monomer is R'CH=CHR" wherein R' and R" are independently hydrogen, hydrocarbyl, fluoroalkyl, or R' and R" may be linked to form a cyclic olefin. Preferred temperature conditions are -100 to 200 °C. Preferred pressure conditions are from about 1 atmosphere to 100 atmospheres.

Examples of processes for preparing polyolefins utilizing **VII** or **VIII** of the third embodiment of the present invention are set forth in the following examples.

### Fourth Embodiment of the Present Invention

The fourth embodiment of the present invention also provides a class of olefin polymerization catalysts based on Group 8-10 transition metal complexes of bidentate ligands comprising one imidate ester, thioimidate ester, selenoimidate ester, or amidine N-donor fragment, and one heterocyclic N-donor fragment, the two fragments together comprising a neutral bidentate, N,N-donor ligand. In one aspect, the catalyst "system" further comprises a Lewis or Bronsted acid.

In a first aspect, the fourth embodiment of the present invention provides a catalyst system comprising a compound of the formula **X**: 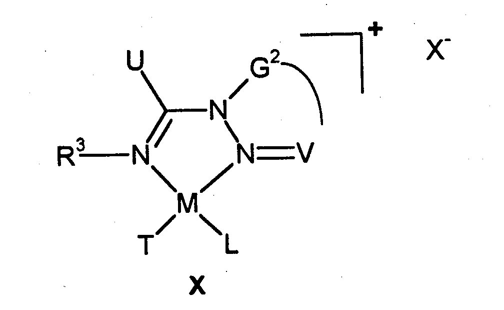 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N;
T is hydrogen, hydrocarbyl, or substituted hydrocarbyl;
L is a monoolefin or a neutral Lewis base where the donating atom is nitrogen, oxygen or sulfur;
M is Ni(II), Pd(II) or Co(II); and
X⁻ is a weakly coordinating anion.

In a second aspect, this fourth embodiment of the present invention provides a catalyst system formed by contacting a first compound Y with a second compound **XI** having the formula: 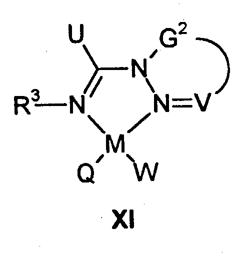 wherein:
R³ is hydrocarbyl, substituted hydrocarbyl or silyl;
U is OR⁴, SR⁴, SeR4 or NR⁴R⁸;
R⁴ and R⁸ are each independently selected from hydrogen, hydrocarbyl, substituted hydrocarbyl, silyl, and in addition, R4 and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N;
M is Ni(II), Pd(II), or Co(II);
Q is hydrocarbyl, chloride, iodide, or bromide;
W is hydrocarbyl, chloride, iodide, or bromide;
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

Preferred neutral Lewis acids include: MAO and other aluminum sesquioxides, R₃⁷Al, R⁷₂AlCl, R⁷AlCl₂ where R⁷ is alkyl. Complex **XI** and compound Y may be combined in the liquid phase. The liquid phase may include solvent or neat monomer. In a process for preparing polyolefins, complex **XI** and compound Y may be combined in the presence of monomer. The molar ratio of compound Y to complex **XI** may be from about 10 to 10,000.

In a third aspect, the fourth embodiment of the present invention provides a compound **XII** of the formula: 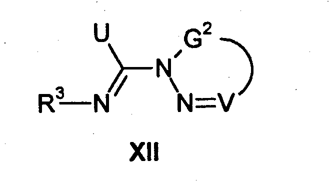 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N.

Compound **XII** may be utilized as a precursor to complex **X** or **XI.**

Complexes **X** and **XI** may be synthesized by a method analogous to that described in the literature (Johnson *et al*., *J. Am. Chem. Soc.* **1995,** *117*, 6414).

The skilled artisan, in possession of this disclosure, could make the present compounds without undue experimentation. Methods of synthesizing complexes **X** and **XI,** and compounds **XII,** are also illustrated in the following examples.

Preferred ligand components for use in complex **X** or **XI** and preferred catalyst systems utilizing complex **X** or **XI** are also set forth in the following examples.

The present invention also provides processes for preparing polyolefins utilizing the complexes **X, XI,** and compound **XII.**

Thus, the present invention provides a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a composition catalyst comprising the formula **X**: 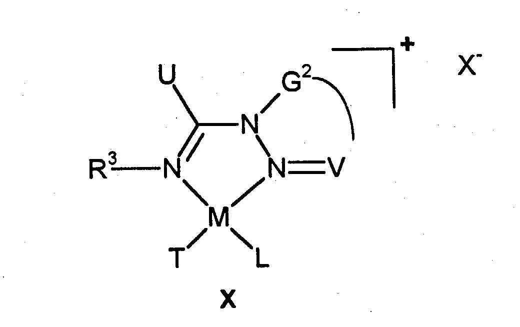 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N;
T is hydrogen, hydrocarbyl, or a substituted hydrocarbyl;
L is a monoolefin or a neutral Lewis base where the donating atom is nitrogen, oxygen, sulfur;
M is Ni(II), Pd(II), or Co(II); and
X⁻ is a weakly coordinating anion;
at a temperature and a pressure sufficient to effect polymerization.

The invention also provides a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst system wherein the catalyst is formed by contacting a first compound Y with a second compound **XI** having the formula: 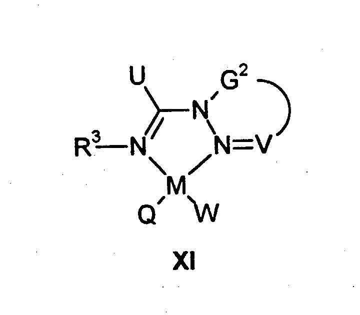 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N;
M is Ni(II), Pd(II), or Co(II);
Q is hydrocarbyl, chloride, iodide, or bromide;
W is hydrocarbyl, chloride, iodide, or bromide; and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion;
at a temperature and a pressure sufficient to effect polymerization.

The invention also provides a process for the polymerization of olefins comprising contacting a compound of the formula **XII** with a suitable nickel precursor, optionally a Lewis or Bronsted acid and one or more olefin monomers of the formula R'CH=CHR" wherein R' and R" are independently hydrogen, hydrocarbyl, fluoroalkyl, or R' and R" may be linked to form a cyclic olefin: 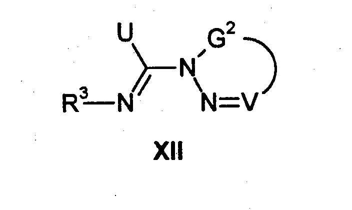 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N.

Preferred polyolefin products will have a degree of polymerization (DP) of at least 10. A preferred olefin monomer is R'CH=CHR" wherein R' and R" are independently hydrogen, hydrocarbyl, fluoroalkyl, or R' and R" may be linked to form a cyclic olefin. Preferred temperature conditions are -100 to 200 °C. Preferred pressure conditions are from about 1 atmosphere to 100 atmospheres.

Examples of processes for preparing polyolefins utilizing complex **X** or complex **XI** of the fourth embodiment of the present invention are set forth in the following examples.

### Fifth Embodiment of the Present Invention

In a fifth embodiment, the present invention provides a catalyst system comprising a transition metal - ligand complex further complexed with one or more Lewis acids to obtain an active olefin polymerization catalyst. Suitable ligands for use in the transition metal-ligand complex comprise the ligand components described above with reference to the second, third and fourth embodiments of the present invention. A preferred transition metal is nickel.

Examples include methylaluminoxane, alkylaluminums, alkylaluminum halides, organoboron compounds, boron halides, B(C₆F₅)₃, Li[BF₄], R₃Sn[BF₄], MgCl₂, M₃SiCl/SnCl₄, SbCl₅, alumina H⁺X⁻, where X⁻ is a relatively non-coordinating anion, and Montmorillonite clay.

An electron deficient metal center is advantageous for efficient olefin polymerization. The fifth embodiment of the present invention provides catalyst systems wherein a binucleating or multinucleating ligand is complexed to a transition metal and one or more Lewis acids to obtain an active olefin polymerization catalyst. While not wishing to be bound by any theory, it is believed that the Lewis acid complexation renders the transition metal more electron deficient rendering the transition metal more active and/or selective, and/or available as a site to bind the catalyst system to a surface. Thus, broadly, catalyst systems of the fifth embodiment of the present invention comprise ligand components, such as the ligand components described with reference to the first, second, third and fourth embodiments of the present invention, complexed with a transition metal and a Lewis acid. Preferred catalyst systems of the fifth embodiment of the present invention include catalyst systems wherein the Lewis acid is bound to one or more heteroatoms which are π-conjugated to the donor atom or atoms bound to the transition metal.

Examples of catalyst systems comprising a Lewis acid - ligand - transition metal complex of the fifth embodiment of the present invention include **XIX**: 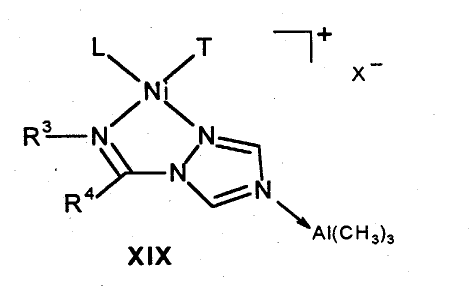 wherein
R3, R4, L, and T are defined as above.

Complex **XIX** may be produced by synthesizing the transition metal - ligand complex in the manner described above and exemplified in the example herein, and then combining the transition metal - ligand complex with the Lewis acid component.

A process of the present invention utilizing the fifth embodiment may be described as follows.

A process for preparing polyolefins comprising contacting one or more olefin monomers with a catalyst system at a temperature and a pressure sufficient to effect polymerization, wherein the catalyst system comprises a transition metal-ligand-Lewis acid complex, e.g. complex **XIX** of the fifth embodiment of the present invention.

Preferred polyolefin products will have a degree of polymerization (DP) of at least 10. A preferred olefin monomer is R'CH=CHR" wherein R' and R" are independently hydrogen, hydrocarbyl, fluoroalkyl, or R' and R" may be linked to form a cyclic olefin. Preferred temperature conditions are -100 to 200 °C. Preferred pressure conditions are from about 1 atmosphere to 100 atmospheres.

A preferred embodiment of the present invention involves a composition comprising (i) a Group 8-10 transition metal M, (ii) one or more Lewis acids, and (iii) a binucleating or multinucleating compound of the formula **XVIII:** 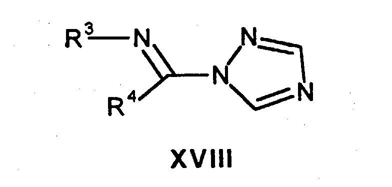 wherein the Lewis acid or acids are bound to one or more heteroatoms which are π-conjugated to the donor atom or atoms bound to the transition metal M;
R³ is hydrocarbyl or substituted hydrocarbyl;
R⁴ is hydrogen, hydrocarbyl, substituted hydrocarbyl, or silyl.

A preferred embodiment of the present invention involves a composition comprising (i) a Group 8-10 transition metal M, (ii) one or more Lewis acids, and (iii) a binucleating or multinucleating compound of the formula **VI**: 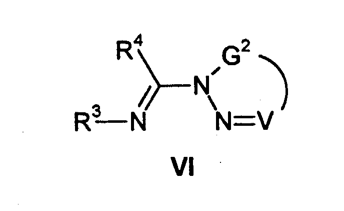 wherein the Lewis acid or acids are bound to one or more heteroatoms which are π-conjugated to the donor atom or atoms bound to the transition metal M;
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus.

A preferred embodiment of the present invention involves a composition comprising (i) a Group 8-10 transition metal M, (ii) one or more Lewis acids, and (iii) a binucleating or multinucleating compound of the formula **IX**: 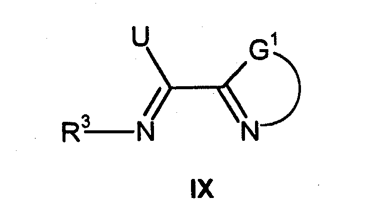 wherein the Lewis acid or acids are bound to one or more heteroatoms which are π-conjugated to the donor atom or atoms bound to a transition metal M;
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N.

A preferred embodiment of the present invention involves a composition comprising (i) a Group 8-10 transition metal M, (ii) one or more Lewis acids, and (iii) a binucleating or multinucleating compound of the formula **XII**: 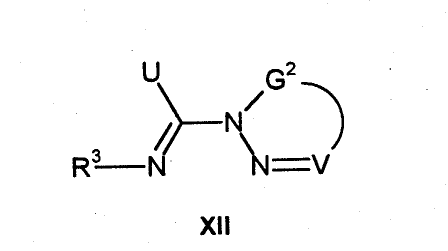 wherein the Lewis acid or acids are bound to one or more heteroatoms which are π-conjugated to the donor atom or atoms bound to a transition metal M;
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N.

The features and advantages of the present invention are further illustrated by the following examples. The procedures described in each example were conducted utilizing conventional laboratory equipment known to those of ordinary skill in the art.

### Ligand Synthesis

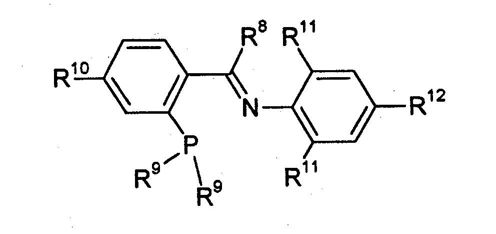

**A1:** R⁸ = H, R⁹ = Ph, R¹⁰ = H, R¹¹ = tBu, R¹² = tBu
**A2:** R⁸ = H, R⁹ = Ph, R¹⁰ = OMe, R¹¹ = iPr, R¹² = H
**A3:** R⁸ = H, R⁹ = Ph, R¹⁰ = H, R¹¹ = iPr, R¹² = H
**A4:** R⁸ = Me, R⁹ = Ph, R¹⁰ = H, R¹¹ = iPr, R¹² = H
**A5:** R⁸ = Ph, R⁹ = Ph, R¹⁰ = H, R¹¹ = iPr, R¹² = H
**A6:** R⁸ = H, R⁹ = Cy, R¹⁰ = H, R¹¹ = iPr, R¹² = H
**A7:** R⁸ = Me, R⁹ = Cy, R¹⁰ = H, R¹¹ = iPr, R¹² = H
**A8:** R⁸ = Ph, R⁹ = Cy, R¹⁰ = H, R¹¹ = iPr, R¹² = H
wherein, H = hydrogen; Ph = phenyl; tBu = t-butyl; OMe = methoxy; iPr = isopropyl; Me = methyl; and Cy = cyclohexyl.

### Example 1

### Synthesis of A1.

Equimolar amounts of diphenylphosphinobenzaldehyde and 2,4,6-tri-*tert*-butylaniline were dissolved in toluene and treated with anhydrous magnesium sulfate and catalytic p-toluenesulfonic acid. The reaction was refluxed overnight. Water was added, and the organic layer was washed successively with saturated sodium bicarbonate and brine. After drying over sodium sulfate, the organic extracts were filtered and concentrated to an oil. Purification by flash chromatography (hexane/EtOAc = 95:5) provided the desired iminophosphine, which was recrystallized from ethanol.

### Example 2

### Synthesis of A2

Equimolar amounts of 2,6-diisopropylaniline and 4-methoxybenzaldehyde were refluxed in ethanol for six hours. Upon cooling, the desired imine precipitated from solution and was recrystallized from ethanol.

A solution of the imine (590 mg) in methanol (6 mL) was added to a suspension of Li₂PdCl₄ (260 mg) in methanol (4 mL). The reaction was stirred at room temperature for 4 hours, at which point the precipitated cyclopalladate complex was isolated by filtration and used without further purification.

A solution of potassium diphenylphosphide (1 mL of 0.5 M solution) in THF (tetrahydrofuran) was added to a mixture of the cyclopalladate (108 mg) and triphenylphosphine (100 mg). After 30 minutes, EtOAc (ethyl acetate) was added to the reaction flask and the solution was washed successively with water and brine, then dried over sodium sulfate and concentrated to a syrup. Purification by flash chromatography (hexane/EtOAc = 99:1) provided the desired iminophosphine **A2** (53 mg).

### Example 3

### Synthesis of A3.

A solution of 2,6-diisopropylaniline (640 µL, 3.39 mmol) and 2-(diphenylphosphino)benzaldehyde (0.98 g, 3.38 mmol) in ethanol (7 mL) was refluxed for six hours. Upon cooling to room temperature, the product crystallized. The yellow crystals were collected by filtration, then recrystallized from ethanol to give the iminophosphine ligand in 69% yield (1.04 g).

### Example 4

### Synthesis of A4.

A cooled solution of 2'-fluoroacetophenone (0.77 mL, 6.25 mmol) and 2,6-diisopropylaniline (2.8 mL, 14.8 mmol) in toluene (4 mL) was treated with TiCl₄ (3 mL of 1.0M solution in toluene). The resulting suspension was allowed to warm to room temperature and stirred overnight. The suspension was filtered through alumina, rinsing with ethyl acetate. The filtrate was concentrated to an oil, which was purified by flash chromatography to yield the imine.

A solution of the imine (172 mg, 0.58 mmol) in ether was added to a solution of potassium diphenylphosphide in THF (1.2 mL of 0.5M solution). After 3 hours, ethyl acetate and water were added. The layers were separated, and the organic layer was washed with brine, dried over sodium sulfate, filtered, and concentrated to an oil. Purification by flash chromatography (Hexane/EtOAc = 98:2) provided the iminophosphine (200 mg, 0.43 mmol) in 74% yield.

### Example 5

### Synthesis of A5.

2-Fluorobenzophenone (0.5 mL) was added to a solution of potassium diphenylphosphide in THF (6 mL of 0.5 M solution). Heat was evolved and the reaction immediately turned green. The reaction went to apparent completion within minutes. After removal of solvent, the residue was dissolved in EtOAc and washed with water and brine, dried over sodium sulfate, filtered, and concentrated. A portion of this material was used directly in the next step to form the imine.

The crude phosphine (about 2/3 of the total material) was treated with 2,6-diisopropylaniline (1.2 mL), dissolved in toluene (6 mL), and cooled in a dry ice/acetone bath. A solution of TiCl₄ (1.5 mL of a 1.0 M solution) in toluene was added. The reaction was allowed to warm to room temperature and stirred for 24 hours. After addition of EtOAc, the suspension was filtered through silica gel, and the filtrate was concentrated, then purified by flash chromatography (Hexane/EtOAc = 98:2) to provide the iminophosphine **A5.**

### Example 6

### Synthesis of A6.

Ethanol (10 mL) was added to 2-bromobenzaldehyde (1.17 mL) and 2,6-diisopropylaniline (1.88 mL) and refluxed for 1 hour. The solution was concentrated, and solidified upon introduction of water. The solid was recrystallized from ethanol to yield the imine.

A solution of butyl lithium in hexanes (1.75 mL of 1.6 M solution, 2.8 mmol) and ether (5 mL) was treated at -78 °C with dicyclohexylphosphine (0.55 mL, 2.75 mmol) and stirred 10 minutes before addition of a solution of the imine (857 mg, 2.49 mmol) in ether (6 mL). The reaction was allowed to warm to room temperature, stirred 18 hours, and worked up using standard extraction techniques. After drying over sodium sulfate, the organic extracts were filtered and concentrated to an oil. Purification by flash chromatography (hexane/EtOAc = 98:2) provided the desired iminophosphine **A6.**

### Example 7

### Synthesis of A7

A cooled solution of acetophenone (4 mmol) and 2,6-diisopropylaniline (10 mmol) in toluene (5 mL) was treated with a solution of TiCl₄ (2 mmol) in toluene (2 mL). After stirring 24 hours, the reaction contents were filtered through alumina, and the filtrate was concentrated, then purified by flash chromatography (hexane/EtOAc = 98:2) to provide the imine.

A suspension of Li₂PdCl₄ and sodium acetate in warm methanol was treated with an equimolar solution of the imine in methanol. After stirring overnight at room temperature, the precipitated cyclopalladate complex was isolated by filtration.

A chilled suspension of the cyclopalladate (174 mg) and triphenylphosphine (200 mg) in ether (2 mL) was treated with a solution of lithium dicyclohexylphosphide in ether/hexanes (0.67 mmol, generated by addition of dicyclohexylphosphine to a solution of BuLi in ether/hexanes). Tetrahydrofuran was also added as a co-solvent. After 24 hours, the reaction contents were filtered through alumina, then concentrated to an oil. Purification by flash chromatography (hexane/EtOAc = 98:2) provided the desired iminophosphine **A7.**

### Example 8

### Synthesis of A8

A cooled solution of benzophenone (4 mmol) and 2,6-diisopropylaniline (10 mmol) in toluene (5 mL) was treated with a solution of TiCl₄ (2 mmol) in toluene (2 mL). After stirring 24 hours, the reaction contents were filtered through alumina, eluting with EtOAc. The filtrate was washed successively with saturated sodium bicarbonate solution, 1 M HCI, and brine. After drying over sodium sulfate, filtration, and concentration, the imine was recrystallized from EtOAc.

A suspension of Li₂PdCl₄ and sodium acetate in warm methanol was treated with an equimolar solution of the imine in methanol. After stirring overnight at room temperature, the precipitated cyclopalladate complex was isolated by filtration.

A chilled suspension of the cyclopalladate (42 mg) and triphenylphosphine (50 mg) in ether (2 mL) was treated with a solution of lithium dicyclohexyl phosphide in ether/hexanes (0.17 mmol, generated by addition of dicyclohexylphosphine to a solution of BuLi in ether/hexanes). Tetrahydrofuran was also added as a co-solvent. After 24 hours, the reaction contents were filtered through alumina, then concentrated to an oil. Purification by flash chromatography (hexane/EtOAc = 98:2) provided the desired iminophosphine **A8**. 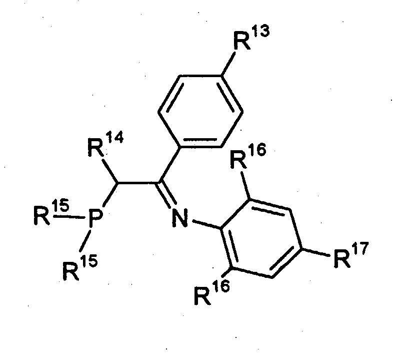
**A9:** R¹³ = H, R¹⁴ = H, R¹⁵ = Ph, R¹⁶ = iPr, R¹⁷ = H
**A10:** R¹³ = CF₃, R¹⁴ = H, R¹⁵ = Ph, R¹⁶ = iPr, R¹⁷ = H
**A11:** R¹³ = H, R¹⁴ = CH₃, R¹⁵ = Ph, R¹⁶ = iPr, R¹⁷ = H
wherein CH₃ = methyl; CF₃ = trifluoromethyl; and the other terms are as described above with reference to **A1 - A8.**

### Example 9

### Synthesis of A9

A cooled solution of acetophenone (4 mmol) and 2,6-diisopropylaniline (12 mmol) in toluene (5 mL) was treated with a solution of TiCl₄ (2 mmol) in toluene (2 mL). After stirring 24 hours, the reaction contents were filtered through alumina, and the filtrate was concentrated, then purified by flash chromatography (hexane/EtOAc = 98:2) to provide the imine.

A solution of the imine in ether was treated with a slight excess of LDA (generated by the addition of BuLi to diisopropylamine) at -78° C and allowed to gradually warm to room temperature over the course of an hour. After cooling the reaction flask in a dry ice/acetone bath, chlorodiphenylphosphine (0.95 eq) was added. After four hours, the reaction contents were filtered through silica gel and concentrated to an oil. The desired β-iminophosphine **A9** is unstable, and was used directly to form the bidentate metal complex with Ni(II).

### Example 10

### Synthesis of A10

A cooled solution of 4'-trifluoromethylacetophenone (5 mmol) and 2,6-diisopropylaniline (15 mmol) in toluene (5 mL) was treated with a solution of TiCl₄ (2.5 mmol) in toluene (2.5 mL). After stirring 24 hours, the reaction contents were filtered through alumina, and the filtrate was concentrated to a solid, which was recrystallized from EtOAc to provide the imine.

A solution of the imine (149 mg, 0.43 mmol) in ether was treated with a slight excess of LDA (0.5 mmol, generated by the addition of BuLi to diisopropylamine) at -78° C and allowed to gradually warm to room temperature over the course of an hour. After cooling the reaction flask in a dry ice/acetone bath, chlorodiphenylphosphine (0.4 mmol) was added. After four hours, the reaction contents were filtered through silica gel and concentrated to an oil. Purification by flash chromatography (hexane/EtOAc = 98:2) provided the desired β-iminophosphine **A10** with trace impurities.

### Example 11

### Synthesis of A11.

A cooled solution of propiophenone (4 mmol) and 2,6-diisopropylaniline (12 mmol) in toluene (5 mL) was treated with a solution of TiCl₄ (2 mmol) in toluene (2 mL). After stirring 24 hours, the reaction contents were filtered through alumina, and the filtrate was concentrated to a syrup, which was purified by flash chromatography (hexane/EtOAc = 98:2) to give the desired imine.

A solution of the imine (195 mg, 0.67 mmol) in ether was treated with a slight excess of LDA (0.75 mmol, generated by the addition of BuLi to diisopropylamine) at -78° C and allowed to gradually warm to room temperature over the course of an hour. After cooling the reaction flask in a dry ice/acetone bath, chlorodiphenylphosphine (0.6 mmol) was added.
After four hours, the reaction contents were filtered through silica gel and concentrated to an oil. The desired product **A11** was not stable to conventional flash chromatography, and thus the residue was used directly to form a catalyst precursor metal complex.

### Synthesis of the Metal Complex

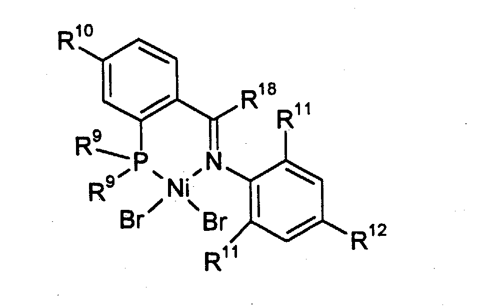

**B1:** R⁸ = H, R⁹ = Ph, R¹⁰ = H, R¹¹ = tBu, R¹² = tBu
**B2:** R⁸ = H, R⁹ = Ph, R¹⁰ = OMe, R¹¹ = iPr, R¹² = H
**B3:** R⁸ = H, R⁹ = Ph, R¹⁰ = H, R¹¹ = iPr, R¹² = H
**B4:** R⁸ = Me, R⁹ = Ph, R¹⁰ = H, R¹¹ = iPr, R¹² = H
**B5:** R⁸ = Ph, R⁹ = Ph, R¹⁰ = H, R¹¹ = iPr, R¹² = H
**B6:** R⁸ = H, R⁹ = Cy, R¹⁰ = H, R¹¹ = iPr, R¹² = H
**B7:** R⁸ = Me, R⁹ = Cy, R¹⁰ = H, R¹¹ = iPr, R¹² = H
**B8:** R⁸ = Ph, R⁹ = Cy, R¹⁰ = H, R¹¹ = iPr, R¹² = H
wherein the terms are as described above with reference to examples 1-11.

### Example 12

### Synthesis of B3

The iminophosphine **A3** (88 mg, 1.96 x 10⁻⁴ mol) and (DME)NiBr₂ (53mg, 1.8 x 10⁻⁴ mol) were combined as solids in an inert atmospheres glove box. The Schlenk flask was removed from the glove box and to the flask was added 10 mL of dry CH₂Cl₂. The deep orange/brown solution was left to stir at 23° C for 6 hours. The solvent was evaporated under reduced pressure resulting in a red/brown powder. The solid was washed with 3 x 15 mL of hexane and then placed under high vacuum to remove all solvent. 64 mg of pre-catalyst **B3** was isolated (70 % yield).

### Example 13

### Synthesis of B4

The iminophosphine **A4** (122 mg, 0.26 mmol) and (DME)NiBr₂ (77mg 0.25mmol) were combined in a Schlenk flask with 20 mL of CH₂Cl₂. The deep brown solution was left to stir at 23° C for 2 days. The solvent was evaporated under reduced pressure resulting in a brown powder. The solid was washed with 3 x 10 mL of diethyl ether and then placed under high vacuum to remove all solvent. 90 mg of pre-catalyst B4 was isolated (53 % yield).

### Example 14

### Synthesis of B5

The iminophosphine **A5** (60 mg, 0.114 mmol) and (DME)NiBr₂ (35mg 0.113mmol) were combined in a Schlenk flask with 15 mL of CH₂Cl₂. The deep brown solution was left to stir at 23° C overnight. The solvent was evaporated under reduced pressure resulting in a brown powder. The solid was washed with 2 x 10 mL of hexane and then placed under high vacuum to remove all solvent. 49 mg of pre-catalyst B5 was isolated (60 % yield). 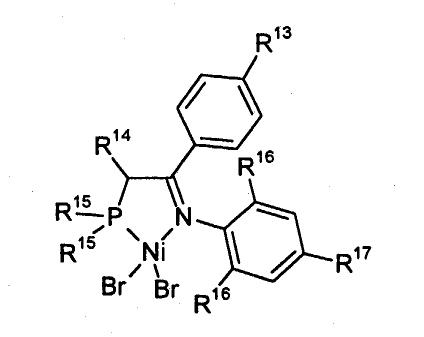
**B9:** R¹³ = H, R¹⁴ = H, R¹⁵ = Ph (phenyl), R¹⁶ = iPr (isopropyl), R¹⁷ = H
**B10:** R¹³ = CF₃, R¹⁴ = H, R¹⁵ = Ph, R¹⁶ = iPr, R¹⁷ = H
**B11:** R¹³ = H, R¹⁴ = CH₃, R¹⁵ = Ph, R¹⁶ = iPr, R¹⁷ = H
wherein all terms are as described with reference to examples 1-11 above.

### Example 15

### Synthesis of B9

A CH₂Cl₂ solution of the iminophosphine **A9** (50 mg, 0.11 mmol) was transferred via cannula onto a suspension of (DME)NiBr₂ (34 mg 0.11 mmol) in 10 mL of CH₂Cl₂. The resulting deep brown solution was left to stir at 23° C for 3 hours. The solvent was evaporated under reduced pressure resulting in a brown powder. The solid was washed with 3 x 10 mL of hexane and then placed under high vacuum to remove all solvent affording **B9.**

### Olefin Polymerization

### Example 16

The iminophosphine complex **B3** (10 mg, 1.5 x 10⁻⁵ mol) was dissolved in 50 mL of dry toluene. The reaction mixture was cooled to 0° C and placed under an ethylene atmosphere. 3.0 mL of (∼ 350 eq) of MAO (10 % by weight in toluene) was added to the polymerization flask. The mixture was left to stir for one hour at room temperature. Acetone, 6M HCI, and H₂O were added to quench the polymerization and precipitate the polyethylene. The polymer was isolated and dried *in vacuo.* ¹H NMR analysis is consistent with branched polyethylene having 57 branches/1000 carbon atoms.

### Example 17

The iminophosphine complex **B4** (15 mg, 2.2 x 10⁻⁵ mol) was dissolved in 50 mL of dry toluene. The reaction mixture was cooled to 0° C and placed under an ethylene atmosphere. 3.0 mL of (∼ 350 eq) of MAO (10 % by weight in toluene) was added to the polymerization flask. The mixture was left to stir for one hour at room temperature. Acetone, 6M HCI, and H₂O were added to quench the polymerization and precipitate the polyethylene. ∼ 50 mg of polymer was isolated and dried *in vacuo.* ¹H NMR analysis is consistent with branched polyethylene and DSC indicates Tₘ = 118° C. GPC: M_{w} = 15,000

### Example 18

The iminophosphine complex **B5** (9 mg, 1.2 x 10⁻⁵ mol) was dissolved in 50 mL of dry toluene. The reaction mixture was cooled to 0° C and placed under an ethylene atmosphere. Approximately 100 eq of MAO (10 % by weight in toluene) was added to the polymerization flask. The mixture was left to stir for one hour at 0° C. Acetone, 6M HCI, and H₂O were added to quench the polymerization and precipitate the polyethylene. The polymer was dried *in vacuo* resulting in 125 mg of polymer. ¹H NMR analysis is consistent with branched polyethylene (35 branches/1000 carbon atoms). DSC: Tₘ = 109° C. GPC: M_{w} = 15,000

### Example 19

The iminophosphine complex **B9** (∼20 mg) was dissolved in 50 mL of dry toluene. The reaction mixture was cooled to 0° C and placed under an ethylene atmosphere. Approximately 200 eq of MAO (10 % by weight in toluene) was added to the polymerization flask. The mixture was left to stir for 20 minutes at 0° C. The mixture was left to stir for an additional 25 minutes at 23° C. Acetone, 6M HCI, and H₂O were added to quench the polymerization and precipitate the polyethylene. The polymer was dried *in vacuo* resulting in 30 mg of polymer. ¹H NMR analysis is consistent with branched polyethylene (40 branches/1000 carbon stoms) and DSC indicates Tₘ = 114° C. GPC: M_{w} = 400,000.

### Ligand Synthesis

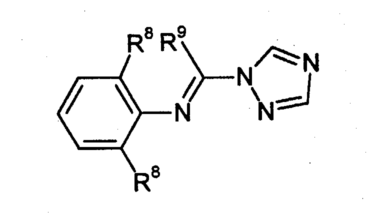

**C1:** R⁸ = Me, R⁹ = 1-naphthyl
**C2:** R⁸ = iPr, R⁹ = 1-naphthyl
**C3:** R⁸ = Me, R⁹ = phenyl
**C4:** R⁸ = iPr, R⁹ = phenyl
**C5:** R⁸ = iPr, R⁹ = 2,6-dimethoxyphenyl

### Example 20

Synthesis of **C1**. Equimolar amounts of 1,2,4-triazole and *N*-(2,6-dimethylphenyl)-1-naphthimidoyl chloride (derived in the conventional fashion [H. Ulrich, *The Chemistry of Imidoyl Halides*, Plenum, New York, 1968] from the corresponding amide and phosphorus pentachloride) were treated with an excess of triethylamine in methylene chloride at room temperature. After 1 hour, the solution was concentrated, taken up in ethyl acetate, filtered, and washed sequentially with saturated sodium bicarbonate solution, 0.5M HCI solution, and brine, then dried over magnesium sulfate, filtered, and concentrated to a syrup. Purification by flash chromatography (hexane/EtOAc = 4:1) afforded the triazole/imine ligand **C1** as a pale yellow solid.

### Example 21

Synthesis of **C2**. A mixture of 1,2,4-triazole (1.2 mmol) and *N*-(2,6-diisopropylphenyl)-1-naphthimidoyl chloride (0.5 mmol) was treated with triethylamine (1 mmol) and methylene chloride (5 mL) and stirred four hours at room temperature. The reaction was worked up as described in example 20 to afford **C2** as a pale yellow solid.

### Example 22

Synthesis of **C3.** A mixture of 1,2,4-triazole (2.9 mmol) and N-(2,6-dimethylphenyl)-1-benzimidoyl chloride (1.5 mmol) was treated with triethylamine (1.5 mmol) and methylene chloride (7 mL) and stirred 14 hours at room temperature. The reaction was worked up as described in example 20 to afford **C3** as a pale yellow solid (242 mg).

### Example 23

Synthesis of **C4.** A mixture of 1,2,4-triazole (1.5 mmol) and N-(2,6-diisopropylphenyl)-1-benzimidoyl chloride (0.7 mmol) was treated with triethylamine (1 mmol) and methylene chloride (6 mL) and stirred overnight at room temperature. The reaction was worked up as described in example 20 to afford **C4** as a pale yellow solid (85 mg) after purification by flash chromatography (hexane/EtOAc = 3:1).

### Example 24

Synthesis of **C5.** 2,6-Diisopropylaniline (25 mmol) was added to a chilled suspension of 2,6-dimethoxybenzoyl chloride (25 mmol) in pyridine (20 mL). After stirring three hours, water (50 mL) was added, preparing a purple precipitate that was isolated by filtration and subsequently recrystallized from ethanol to provide the amide as a purple solid.

A solution of the amide (690 mg, 2 mmol) in acetonitrile (8 mL) was added to a mixture formed by the addition of triethylamine (580 µL) to a cooled suspension of 1,2,4-triazole (420 mg, 6 mmol) and POCl₃ in acetonitrile (8 mL). After stirring 24 hours at room temperature, the reaction contents were filtered, and the filtrate was evaporated to an oil. The oil was dissolved in EtOAc, and washed successively with aqueous solutions of sodium bicarbonate, HCI, and NaCI. The organic layer was dried over magnesium sulfate, filtered, and concentrated to an oil. Purification by flash chromatography (hexane/EtOAc = 3:1) afforded the desired imino-substituted heterocyclic **C5**. 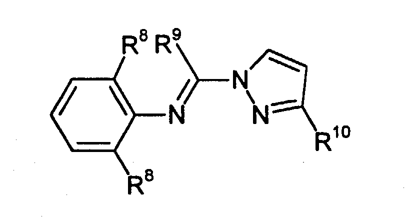
**C6:** R⁸ = Me, R⁹ = 1-naphthyl, R¹⁰ = CH₃
**C7:** R⁸ = iPr, R⁹ = 1-naphthyl, R¹⁰ = CH₃
**C8:** R⁸ = Me, R⁹ = 1-naphthyl R¹⁰ = H

### Example 25

Synthesis of **C6.** A mixture of 3-methylpyrazole (0.8 mmol) and *N*-(2,6-dimethylphenyl)-1-naphthimidoyl chloride (0.8 mmol) was treated with triethylamine (1.5 mmol) and methylene chloride (5 mL) and stirred 4 hours at room temperature. The reaction was worked up as described in example 20 to afford the desired pyrazole/imine **C6.**

### Example 26

Synthesis of **C7.** A mixture of 3-methylpyrazole (1.0 mmol) and *N*-(2,6-diisopropylphenyl)-1-naphthimidoyl chloride (0.7 mmol) was treated with triethylamine (1.0 mmol) and methylene chloride (5 mL) and stirred 1 hour at room temperature. The reaction was worked up as described in example 20 to afford the desired pyrazolelimine **C7.**

### Example 27

Synthesis of **C8.** A mixture of pyrazole (4.0 mmol) and *N*-(2,6-dimethylphenyl)-1-naphthimidoyl chloride (1.5 mmol) was treated with triethylamine (2.0 mmol) and methylene chloride (8 mL) and stirred 16 hours at room temperature. The reaction was worked up as described in example 20 to afford the desired pyrazole/imine **C8** as pale yellow crystals. 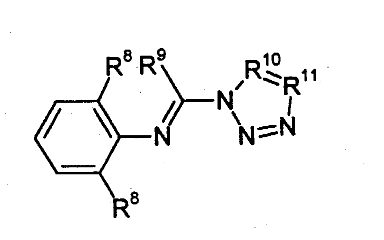
**C9:** R⁸ = iPr; R⁹ = 1-naphthyl; R¹⁰, R¹¹ = C
**C10:** R⁸ = iPr; R⁹ = phenyl; R¹⁰, R¹¹ = C-Ph and N (one C, one N)

### Example 28

Synthesis of **C9.** A mixture of 1*H*-1,2,3-triazole (100 µL) and *N*-(2,6-diisopropylphenyl)-1-naphthimidoyl chloride (200 mg) was treated with triethylamine (150 µl) and methylene chloride (6 mL) and stirred 1 hour at room temperature. The reaction was worked up as described in example 20 to afford the desired triazolelimine **C9.**

### Example 29

Synthesis of **C10.** A mixture of 5-phenyl-1*H*-tetrazole (160 mg, 1.1 mmol) and *N*-(2,6-diisopropylphenyl)-1-benzimidoyl chloride (0.7 mmol) was treated with triethylamine (250 µl) and methylene chloride (6 mL) and stirred 16 hours at room temperature. The reaction was worked up as described in example 20. Attempts to further purify the desired product using silica gel chromatography led to ligand decomposition, and thus the crude product of the workup was used directly in the next step to form the nickel complex **D10**. 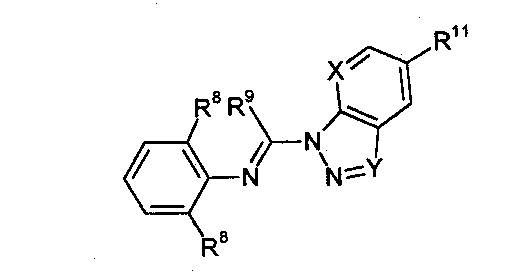
**C11:** R⁸ = iPr; R⁹ = phenyl; R¹¹ = COOH; X = CH; Y = N
**C12:** R⁸ = iPr; R⁹ = phenyl; R¹¹ = H; X = N; Y = N
**C13:** R⁸ = iPr; R⁹ = CF₃; R¹¹ = H; X = CH; Y = CH
**C14:** R⁸ = iPr; R⁹ = CF₃; R¹¹ = NO₂; X = CH; Y = CH

### Example 30

Synthesis of **C11.** A mixture of benzotriazole-5-carboxylic acid (163 mg, 1.0 mmol) and *N*-(2,6-diisopropylphenyl)-1-benzimidoyl chloride (0.7 mmol) was treated with triethylamine (260 µl) and methylene chloride (6 mL) and stirred overnight at room temperature. The reaction was worked up as described in example 20 and purified by flash chromatography (hexane/ethyl acetate = 3:1) to afford the desired benzotriazole/imine **C11,** as well as a contaminant byproduct (MS = 689) which may be the diacylated product resulting from acylation of both the desired ring nitrogen and the carboxylic acid.

### Example 31

Synthesis of **C12** A mixture of 1*H*-1,2,3-triazolo(4,5-β)pyridine (1.0 mmol) and *N*-(2,6-diisopropylphenyl)-1-benzimidoyl chloride (0.5 mmol) was treated with triethylamine (1.0 mmol) and methylene chloride (7 mL) and stirred 16 hours at room temperature. The reaction was worked up as described in example 20 and used directly without further purification.

### Example 32

Synthesis of **C13** A solution of indazole (202 mg, 1.71 mmol) in THF (5.0 mL) was cooled to 0 °C in an ice bath and treated with NaH (60% dispersion in mineral oil, 106 mg, 2.66 mmol). The resulting suspension was stirred at 0 °C for 25 min, then treated with N-(2,6-diisopropylphenyl)-trifluoroacetimidoyl chloride (497 mg, 1.70 mmol) [which had been prepared according to the procedure of K. Tamura, et al., *J. Org. Chem.* **1993,** *58*, 32-35, from trifluoroacetic acid, 2,6-diisopropyl aniline, carbon tetrachloride, triphenylphosphine and triethylamine] via syringe, rinsing the syringe with THF (0.5 mL). The ice bath was removed, and the suspension allowed to stir at rt for 1.5h. The solvent was removed *in vacuo*, and the residue was partitioned between saturated NaHCO₃ (5 mL) and CH₂Cl₂ (5 mL). The aqueous layer was further extracted with CH₂Cl₂ (2 X 5 mL). The combined organic extracts were dried over Na₂SO₄, filtered and concentrated in vacuo. The resulting residue was flash chromatographed (SiO₂, 4% EtOAc : Hex) to afford **C13** (548 mg, 86%): R_{f} 0.27 (4% EtOAc : Hex); ¹H-NMR (300 MHz, CDCl₃) δ 8.45 (d, 1 H, *J* = 8.2 Hz), 8.29 (d, 1 H, *J* = 0.6 Hz), 7.84 (dt, 1 H, *J* = 8.0 Hz, *J* = 1.1 Hz), 7.54 (ddd, 1H, *J* = 8.4 Hz, *J* = 7.1 Hz, *J* 1.1 Hz), 7.40 (ddd, 1 H, *J* = 8.0 Hz, *J* = 7.1 Hz, *J* = 0.8 Hz), 7.06-7.20 (m, 3H), 2.85 (p, 2H, *J* = 6.9 Hz), 1.20 (d, 6H, *J* = 6.9 Hz), 1.15 (d, 6 H, *J* = 6.6 Hz); IR (film) 2965, 1684, 1431, 1171, 1154, 926 cm⁻¹; FDMS *m*/*z* 373 (M+, 100%).
**C13** was contaminated with a small amount of unidentified impurities: ¹H NMR (300 MHz, CDCl₃) δ 7.72 (d, *J* = 9.1 Hz), 2.72 (d, *J* = 6.9 Hz).

### Example 33

Synthesis of **C14** A solution of 5-nitroindazole (286 mg, 1.75 mmol) in THF (5 mL) was cooled to 0 °C in an ice bath and treated with NaH (60% dispersion in mineral oil, 110 mg, 2.74 mmol). The resulting suspension was stirred at 0 °C for 20 min, then treated with N-(2,6-diisopropylphenyl)-trifluoroacetimidoyl chloride (509 mg, 1.74 mmol) via syringe, rinsing the syringe with THF (0.5 mL). The ice bath was removed, and the suspension stirred at rt for 2 h. The solvent was removed *in vacuo*, and the residue partitioned between saturated NaHCO₃ (5 mL) and CH₂Cl₂ (5 mL). The aqueous layer was further extracted with CH₂Cl₂ (2 X 5 mL). The combined organic extracts were dried over Na₂SO₄, filtered and concentrated *in vacuo.* The residue was flash chromatographed (3% EtOAc : Hex) to afford **C14** (573 mg, 78%): R_{f} 0.16 (3% EtOAc : Hex); ¹H NMR (300 MHz, CDCl₃) δ 8.80 (dd, 1 H, *J* = 2.2 Hz, *J* = 0.5 Hz), 8.47 (s, 1H), 8.42 (dd, 1H, *J* = 9.2 Hz, *J* = 1.9 Hz), 8.12 (dd, 0.5 H, *J* = 9.8 Hz, *J* = 1.9 Hz), 7.85 (d, 0.5 H, *J* = 9.6 Hz), 7.18-7.22 (m, 3 H), 2.79 (p, 2 H, *J* = 6.9 Hz), 1.20 (br d, 6 H, *J* = 5.8 Hz), 1.16 (br d, 6 H, *J* = 6.0 Hz); IR (Film) 2965, 1686, 1528, 1431, 1345, 1172, 1150, 922; FDMS *m*/*z* 418 (M+, 100%).
**C14** was contaminated with a small amount of unidentified impurities: ¹H NMR (300 MHz, CDCl₃) δ 8.52-8.64 (br m), 2.69 (p, *J* = 6.9 Hz), 0.85-1.10 (br s). 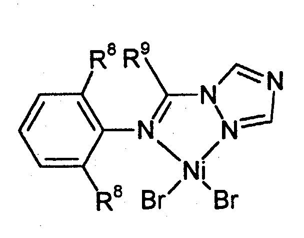
**D1:** R⁸ = Me, R⁹ = 1-naphthyl
**D2:** R⁸ = iPr, R⁹ = 1-naphthyl
**D3:** R⁸ = Me, R⁹ = phenyl
**D4:** R⁸ = iPr, R⁹ = phenyl
**D5:** R⁸ = iPr, R⁹ = 2,6-dimethoxyphenyl

### Example 34

Synthesis of **D1.** A methylene chloride solution of the triazolelimine **C1** (46 mg, 0.14 mmol) and (DME)NiBr₂ (37 mg, 0.12 mmol) were combined via stainless steel cannula. The mixture was left to stir at 23 °C for 16 hours. The solvent was partially evaporated under reduced pressure and 10 mL of hexane was added to fully precipitate the complex. The resulting yellow solid was washed with hexane and then placed under high vacuum to remove all solvent. 37 mg of pre-catalyst **D1** isolated (60 % yield).

### Example 35

Synthesis of **D2.** A flame dried Schlenk flask equipped with a magnetic stir bar and capped with a rubber septum. To the flask was added DMENiBr₂ (59 mg, 0.191 mmol) and 10 mL of methylene chloride. In a separate flask the imine/triazole **C2** (78 mg, 0.204mmol) was dissolved in 5 mL of methylene chloride and transferred via stainless steel canula onto the DMENiBr₂ suspension. The mixture was stirred at room temperature for 16 hours. After 16 hours, the methylene chloride was removed *in vacuo* resulting in a yellow/green solid. The solid was washed with 2 X 10 mL of hexane. The solid was left to dry under reduced pressure for several hours resulting in 42 mg of the imine/triazole complex **D2.**

### Example 36

Synthesis of **D3.** Methylene chloride (8 mL) was added to a mixture of triazole/imine **C3** (54 mg) and (DME)NiBr₂ (57 mg). The reaction was stirred at room temperature overnight. Solvent was removed via filter cannula, and the pale yellow powder was washed with hexane, then dried *in vacuo.*

### Example 37

Synthesis of **D4.** Methylene chloride (6 mL) was added to a mixture of triazole/imine **C4** (60 mg) and (DME)NiBr₂ (45 mg). The reaction was stirred at room temperature overnight. The solvent was partially evaporated under a stream of argon, and hexane was added. The precipitated pale green complex was washed several times with hexane, then dried *in vacuo.*

### Example 38

Synthesis of **D5.** Methylene chloride (9 mL) was added to a mixture of ligand **C5** (125 mg) and (DME)NiBr₂ (83 mg). The reaction was stirred at room temperature overnight. The solvent was partially evaporated under a stream of argon, and hexane was added. The precipitated pale green complex was washed several times with hexane, then dried *in vacuo.* 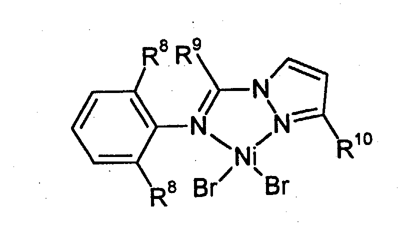
**D6:** R⁸ = Me, R⁹ = 1-naphthyl, R¹⁰ = CH₃
**D7:** R⁸ = iPr, R⁹ = 1-naphthyl, R¹⁰ = CH₃

### Example 39

Synthesis of **D6.** Methylene chloride (10 mL) was added to a mixture of the pyrazole/imine ligand **D6** (81 mg, 0.24 mmol) and (DME)NiBr₂ (60 mg, 0.19 mmol). The suspension immediately turned a deep orange/brown color, and was allowed to stir 18 hours. The solvent was evaporated under a stream of argon, and the precipitated orange solid was washed several times with hexane and hexane/methylene chloride before drying *in vacuo.* The pre-catalyst **F3** was isolated as an orange/brown powder.

### Example 40

Synthesis of **D7.** A flame dried Schlenk flask equipped with a magnetic stir bar and capped with a rubber septum. To the flask was added DMENiBr₂ (71 mg, 0.23 mmol) and 10 mL of methylene chloride. In a separate flask the imine/heterocyclic **C7** (98 mg, 0.25mmol) was dissolved in 5 mL of methylene chloride and transferred via stainless steel canula onto the DMENiBr₂ suspension. The mixture was stirred at room temperature for 16 hours. After 16 hours, the methylene chloride was removed *in vacuo* resulting in a red/brown solid. The solid was washed with 2 X 5 mL of hexane. The solid was left to dry under reduced pressure for several hours resulting in 97 mg of the imine/heterocycle complex **D7.**

### Synthesis of

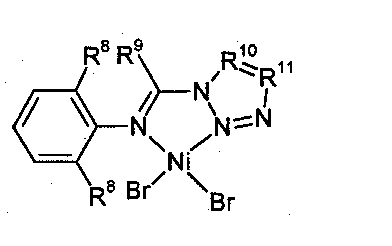

**D9:** R⁸ = iPr; R⁹ = 1-naphthyl; R¹⁰, R¹¹ = C
**D10:** R⁸ = iPr; R⁹ = phenyl; R¹⁰, R¹¹ = C-Ph and N (one C, one N)

### Example 42

Synthesis of **D9.** Methylene chloride (5 mL) was added to a mixture of the triazole/imine ligand **C9** (42 mg) and (DME)NiBr₂ (200 mg). The suspension was allowed to stir 18 hours, at which time the solvent was evaporated under a stream of argon, and the precipitated yellow-orange solid was washed several times with hexane and hexane/methylene chloride before drying *in vacuo.*

### Example 43

Synthesis of **D10.** Methylene chloride (7 mL) was added to a mixture of the tetrazole/imine ligand **C10** (82 mg) and (DME)NiBr₂ (40 mg). The suspension was allowed to stir 18 hours, at which time the solvent was evaporated under a stream of argon, and the precipitate pale green solid was washed several times with hexane and hexane/methylene chloride before drying *in vacuo.* 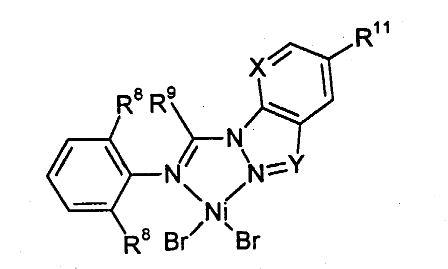
**D11:** R⁸ = iPr; R⁹ = phenyl; R¹¹ = COOH, X = CH; Y = N
**D12:** R⁸ = iPr; R⁹ = phenyl; R¹¹ = H; X = N; Y = N
**D13:** R⁸ = iPr; R⁹ = CF₃; R¹¹ = H; X = CH; Y = CH
**D14:** R⁸ = iPr; R⁹ = CF₃; R¹¹ = NO₂; X = CH; Y = CH

### Example 44

Synthesis of **D11.** Methylene chloride (5 mL) was added to a mixture of excess benzotriazole/imine ligand **C11** and (DME)NiBr₂. The reaction was allowed to stir 18 hours, at which time the solvent was evaporated under a stream of argon, and the precipitated beige solid was washed several times with hexane and hexane/methylene chloride before drying *in vacuo.*

### Example 45

Synthesis of **D12.** Methylene chloride was added to a mixture of the benzotriazole/imine ligand **C12** (82 mg) and a deficiency of (DME)NiBr₂. A green precipitate appeared almost immediately. The solvent was evaporated under a stream of argon, and the precipitated pale green solid was washed several times with hexane and hexane/methylene chloride before drying *in vacuo.*

### Example 46

Synthesis of **D13** A mixture of imine/indazole adduct **C13** (112 mg, 0.299 mmol) and (DME)NiBr₂ (73 mg, 0.238 mmol) was dissolved in CH₂Cl₂ (10 mL). The resulting solution was stirred at rt overnight. The suspension was concentrated under a stream of Ar, and the resulting precipitate was washed several times with hexanes. The precipitate was dried *in vacuo* to afford dibromo complex **D13.**

### Example 47

Synthesis of **D14** A mixture of imine/indazole adduct **C14** (100 mg, 0.238 mmol) and (DME)NiBr₂ (63 mg, 0.206 mmol) was dissolved in CH₂Cl₂ (10 mL), and stirred at rt overnight. The orange/yellow suspension was concentrated under a stream of Ar, and washed with hexanes (10 mL). The residue was dried *in vacuo* to afford **D14** as an orange solid.

### POLYMERIZATIONS

### Example 48

The triazole/imine complex **D1** (2.0 mg) was suspended in 50 mL of dry toluene. The reaction mixture was equilibrated at room temperature under an ethylene atmosphere, then treated with MAO (2.0 mL of a 10 % by weight solution in toluene) and stirred under 1 atm ethylene. A white polyethylene precipitate was observed within minutes. After five minutes, the reaction was quenched by the sequential addition of acetone, methanol, and 6M HCI. The precipitate was isolated by filtration, washed, and dried *in vacuo* to yield 700 mg of polyethylene (85,000 To/h). DSC: Tₘ = 123. GPC: M_{w} = 23,000.

### Example 49

The triazole/imine complex **D3** (2.7 mg) was suspended in 20 mL of dry toluene. The reaction mixture was equilibrated at 0°C under an ethylene atmosphere, then treated with MAO (2.0 mL of a 10 % by weight solution in toluene) and stirred under 1 atm ethylene. After 15 minutes, the reaction was quenched by the sequential addition of acetone, methanol, and 6M HCl. The precipitate was isolated by filtration, washed, and dried *in vacuo* to yield 747 mg of polyethylene. (19,600 To/h, Mn = 6900, Mw = 52,200 (GPC); 2 branches/1000 carbons by NMR)

### Example 50

The pyrazole/imine complex **D6** (14.6 mg) was suspended in 18 mL of dry toluene. The reaction mixture was cooled to 0°C and placed under an ethylene atmosphere. A 10% by weight solution of MAO in toluene (1.8 mL) was added to the polymerization flask. The ice bath was removed and the mixture was left to stir for thirty minutes with substantial evolution of heat. Acetone, 6M HCI, and H₂O were added to quench the polymerization and precipitate the polyethylene. The polymer was isolated by filtration, washed, and dried *in vacuo.* The procedure was repeated without removing the ice bath, with similar polymer characteristics. In both cases, ¹H NMR analysis was consistent with branched polyethylene of relatively low molecular weight (<5,000).

### Example 51

The triazole/imine complex **D9** (4.0 mg) was suspended in 18 mL of dry toluene. The reaction mixture was cooled to 0°C and placed under an ethylene atmosphere. A 10% by weight solution of MAO in toluene (1.8 mL) was added to the polymerization flask. The mixture was left to stir for 45 minutes at 0°C. Acetone, 6M HCI, and H₂O were added to quench the polymerization. No precipitated polyethylene was observed. Separation and subsequent evaporation of the aqueous layer yielded a small amount of solid. The reaction was repeated at room temperature, and a small amount of amorphous material was recovered.

### Example 52

A 250 mL flame dried Schlenk flask was charged with bis(1,5-cyclooctadiene)nickel(0) (14.0 mg, 0.051 mmol), HBAr₄ (Ar = 3,5-bis(trifluoromethyl)phenyl) (39.4 mg, 0.046 mmol) and imine/indazole adduct **C13** (13.7 mg, 0.037 mmol). The flask was evacuated and backfilled with ethylene, then charged with toluene (30 mL) resulting in the formation of a dark green/blue solution. The reaction exothermed to ∼ 45 °C, and was allowed to stir under ethylene (1 atm) with no temperature control for 1.5 h, after which it was quenched by the addition of acetone and MeOH. The solvent was removed *in vacuo* to afford an oily waxy solid (2.83g): GPC: Mₙ = 327; M_{w}/Mₙ = 9; ¹H NMR: 92 branches/1000 carbon atoms.

### Example 53

A 250 mL flame dried Schlenk flask was charged with bis(1,5-cyclooctadiene)nickel(0) (8.5 mg, 0.031 mmol), HBAr₄ (Ar = 3,5-bis(trifluoromethyl)phenyl) (24.3 mg, 0.028 mmol) and imine/indazole adduct **C14** (11.0 mg, 0.026 mmol). The flask was evacuated and backfilled with ethylene, then charged with toluene (30 mL) resulting in the formation of an orange/brown solution. The reaction was allowed to stir under ethylene (1 atm) at rt for ~ 1.33 h, after which it was quenched by the addition of acetone and MeOH. The resulting polyethylene was collected by vacuum filtration, and dried *in vacuo* to afford a white polyethylene (117.2 mg): GPC: Mₙ = 5710; M_{w}/Mₙ = 4.7; ¹H NMR: 42 branches/1000 carbon atoms.

### Example 54

A flame dried Schlenk flask was charged with complex **D7** (6 mg, 0.0098 mmol) and 50 mL of toluene. The flask was then cooled to 0 C in an ice bath. MAO (1.5 mL of a 10 wt.% solution in toluene) was then added to the suspension and the reaction left to stir for 30 minutes. Acetone, methanol and 6M HCI were added to quench the reaction and precipitate the resulting branched polyethylene (¹H NMR Mₙ = 2500).

### Example 55

A flame dried Schlenk flask was charged with complex **D1** (5 mg, 0.0092 mmol), norbornene (2g) and 50 mL of toluene. The flask was then placed in a water bath (23 C) to control reaction temperatue. MAO (2 mL of a 10 wt.% solution in toluene) was then added to the suspension and the reaction left to stir for 16 hours. Acetone and methanol were added to quench the reaction and precipitate the resulting polynorbornene. The polymer was collected by filtration and washed with 6M HCI, water, and acetone. The polymer was dried in a vacuum oven resulting in 700 mg of polynorbornene. GPC: Mₙ = 14,500; M_{w} = 44,000.

### Example 56

A flame dried Schlenk flask was charged with complex **D4** (6 mg, 0.0091 mmol), 1 atmosphere ethylene and 50 mL of toluene. The flask was then placed in a water bath (23 C) to control reaction temperatue. MAO (2 mL of a 10 wt.% solution in toluene) was then added to the suspension and the reaction left to stir for 20 minutes (polymer began to precipitate within minutes). Acetone, methanol and 6M HCI were added to quench the reaction and precipitate the resulting polyethylene. The polymer was dried in a vacuum oven resulting in 400 mg of polyethylene. GPC: Mₙ = 2100; M_{w} = 5600. ¹H NMR: 30 branches/1000 carbon atoms. DSC: Tₘ = 104 C.

### Example 57

A flame dried Fisher-Porter bottle was charged with complex **D2** (5 mg, 0.0083 mmol) and 50 mL of toluene. The flask was placed in a water bath (23 C) to control reaction temperatue. MAO (2 mL of a 10 wt.% solution in toluene) was then added to the suspension and the bottle rapidly pressurized to 45 psig and the reaction left to stir for 20 minutes. Acetone, methanol and 6M HCI were added to quench the reaction and precipitate the resulting polyethylene. The polymer was dried in a vacuum oven resulting in 150 mg of polyethylene. GPC: M_{w} = 22,000. ¹H NMR: 7 branches/1000 carbon atoms. DSC: Tₘ = 127 C.

### Example 58

A flame dried Schlenk flask was charged with complex **D4** (2 mg, 0.0036 mmol), 1 atmosphere ethylene and 50 mL of toluene. The flask was then placed in an ice-water bath (0 C) to control reaction temperatue. MAO (2 mL of a 10 wt.% solution in toluene) was then added to the suspension and the reaction left to stir for 10 minutes (polymer began to precipitate within minutes). Acetone, methanol and 6M HCI were added to quench the reaction and precipitate the resulting polyethylene. The polymer was dried in a vacuum oven resulting in 560 mg of polyethylene (33,000 TO/h). GPC: Mₙ = 5500; M_{w} = 27,000. ¹H NMR: 4 branches/1000 carbon atoms. DSC: Tₘ = 130 C.

### Example 59

A flame dried Schlenk flask was charged with complex **D4** (2 mg, 0.0036 mmol), 1 atmosphere ethylene and 50 mL of toluene. The flask was then placed in a water bath (23 C) to control reaction temperatue. MAO (2 mL of a 10 wt.% solution in toluene) was then added to the suspension and the reaction left to stir for 10 minutes (polymer began to precipitate within minutes). Acetone, methanol and 6M HCI were added to quench the reaction and precipitate the resulting polyethylene. The polymer was dried in a vacuum oven resulting in 490 mg of polyethylene (30,000 TO/h). GPC: Mₙ = 1600; M_{w} = 6100. ¹H NMR: 20 branches/1000 carbon atoms. DSC: Tₘ = 110 C.

### Ligand Synthesis

### Example 60

This example illustrates the preparation of a compound IV having the formula: 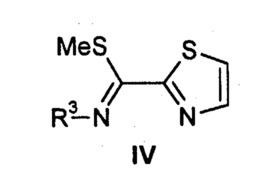 for use in a catalyst system according to the third embodiment of the present invention.
2-Thiazolecarboxylic acid (0.1 mol) is reacted with 1,1-carbonyldiimidazole (0.1 mol) and 2,6-diisopropylaniline (0.1 mol) to obtain the corresponding amide. This is reacted with Lawesson's reagent to form the thioamide, which is treated with Mel and base to give compound **IV.**

### Synthesis of the Metal Complex

### Example 61

This example illustrates the synthesis of a metal complex having the formula **V:** 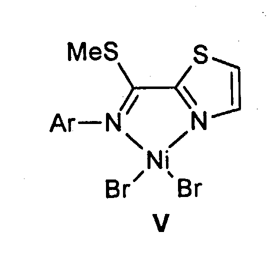 wherein: Ar = 2,6-diisopropylphenyl.

A 50 mL Schlenk flask equipped with a magnetic stir bar and capped with a septum is charged with 0.2 mmol of compound **IV** from example 54 and 0.2 mmol (1,2-dimethoxyethane)nickel(ll) dibromide (Aldrich) under an inert atmosphere. Dry, deoxygenated dichloromethane (5 mL) is added and the mixture is stirred under an argon atmosphere, slowly preparing a crystalline precipitate. After 1 h, another 5 mL dichloromethane is added. The mixture is stirred another 21 h at 21 C, then diluted with 10 mL dry, deoxygenated hexane and stirred another 8 h. The supernatant is removed via a filter paper-tipped cannula, and the residue dried in vacuo at 1 mm Hg to afford complex **V.**

### Olefin Polymerization

### Example 62

A 200 mL pear-shaped Schlenk flask equipped with a magnetic stir bar and capped with a septum is charged with 10 mg of compound **V**. The flask is evacuated and refilled with ethylene, then charged with 75 mL dry, deoxygenated toluene. The resultant suspension is cooled to 0°C and allowed to equilibrate with 1 atm ethylene for 15 min, then treated with 4.0 mL of a 10 wt% solution of MAO in toluene (Aldrich) and stirred under 1 atm ethylene. After 60 minutes, the reaction is quenched by the addition of methanol, acetone and 6 N aqueous HCI to produce a polyethylene precipitate.

### Ligand Synthesis

### Example 63

This example illustrates the preparation of a compound **VI** having the formula: 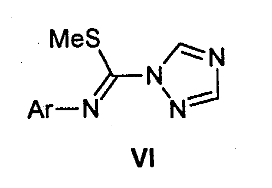 wherein: Ar = 2,6-diisopropylphenyl,
for use in a catalyst system according to the third embodiment of the present invention.

Thiophosgene (0.1 mol) is reacted with 1,2,4-triazole (0.2 mol) and base to afford thiocarbonylditriazole. This is heated with 2,6-diisopropylaniline (0.1 mol) to obtain the mixed thiourea, which is treated with Mel (methyl-iodine) and base to give compound **VI**.

### Synthesis of the Metal Complex

### Example 64

This example illustrates the synthesis of a metal complex having the formula **VII**: 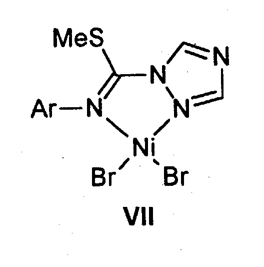 wherein: Ar = 2,6-diisopropylphenyl.

### Example 65

A 50 mL Schlenk flask equipped with a magnetic stir bar and capped with a septum is charged with 0.2 mmol of compound **VI** from example 57 and 0.2 mmol (1,2-dimethoxyethane)nickel(ll) dibromide (Aldrich) under an inert atmosphere. Dry, deoxygenated dichloromethane (5 mL) is added and the mixture is stirred under an argon atmosphere, slowly preparing a crystalline precipitate. After 1 hour, another 5 mL dichloromethane is added. The mixture is stirred another 21 hour at 21° C, then diluted with 10 mL dry, deoxygenated hexane and stirred another 8 hour. The supernatant is removed via a filter paper-tipped cannula, and the residue dried *in vacuo* at 1 mm Hg to afford compound **VII.**

### Olefin Polymerization

### Example 66

A 200 mL pear-shaped Schlenk flask equipped with a magnetic stir bar and capped with a septum is charged with 10 mg of compound **VII** from example 58. The flask is evacuated and refilled with ethylene, then charged with 75 mL dry, deoxygenated toluene. The resultant suspension is cooled to 0°C and allowed to equilibrate with 1 atm ethylene for 15 min, then treated with 4.0 mL of a 10 wt% solution of MAO in toluene (Aldrich) and stirred under 1 atm ethylene. After 60 minutes, the reaction is quenched by the addition of methanol, acetone and 6 N aqueous HCI to produce a polyethylene precipitate.

### Ligand Synthesis

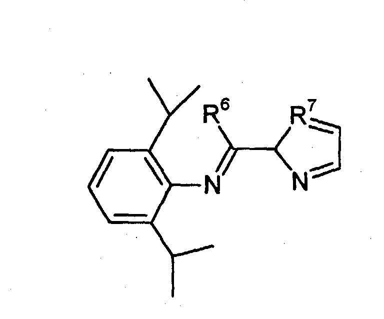

**E1:** R⁶ = Me, R⁷ = S
**E2:** R⁶ = H, R⁷ = N(H)

### Example 67

### Synthesis of E1

A cooled solution of 2-acetylthiazole (4 mmol) and 2,6-diisopropylaniline (12 mmol) in toluene (15 mL) is treated with TiCl₄ (2.0 mL of 1.0 M solution in toluene), immediately yielding an olive green precipitate. After allowing the reaction to warm to room temperature, stirring is continued for 72 hours before the reaction contents are filtered through alumina, rinsing with ethyl acetate. The filtrate is washed sequentially with 0.6 M HCI, saturated sodium bicarbonate, and brine, then dried over magnesium sulfate, filtered, and concentrated to afford **E1** as a yellow solid.

### Example 68

### Synthesis of E2

2-lmidazolecarboxaldehyde (2.9 mmol) is added to a solution of diisopropylaniline (2.9 mmol) in ethanol (10 mL). The reaction is refluxed for 4 hours, then concentrated to a small volume, taken up in ethyl acetate, and washed. Removal of solvent by rotary evaporation gives an oil that crystallized over time, is isolated by filtration, washed, and used without further purification.

### Synthesis of the Metal Complex

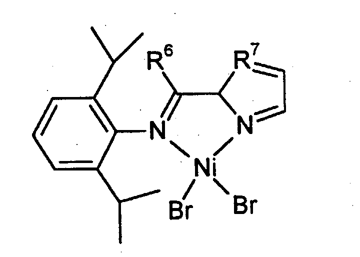

**F1:** R⁶ = Me, R⁷ = S
**F2:** R⁶ = H, R⁷ = N(H)

### Example 69

### Synthesis of F1.

A molar excess of thiazole/imine **E1,** from Example 60, and (DME)NiBr₂ are combined as solids in an inert atmospheres glove box, then removed from the glove box and treated with dry CH₂Cl₂. The suspension immediately develops a deep orange/brown color and is stirred overnight at room temperature. The solvent is evaporated under a stream of argon, and the precipitated solid is washed several times with hexane and hexane/methylene chloride before being dried *in vacuo* to give the pre-catalyst **F1** as an orange/brown powder.

### Example 70

### Synthesis of F2.

Methylene chloride (10 mL) is added to a mixture of crude imidazole/imine **E2** (100 mg), from example 61, and (DME)NiBr₂ (27 mg). The suspension gradually develops a bright green color, then appears to darken somewhat. After stirring overnight, the solvent is evaporated under a stream of argon, and the precipitated solid is washed several times with hexane and hexane/methylene chloride before being dried *in vacuo* to give the pre-catalyst **F2** as a yellow-green powder.

### Olefin Polymerization

### Example 71

The thiazole/imine complex **F1** (12 mg), from Example 62, is suspended in 20 mL of dry toluene. The reaction mixture is cooled to 0°C and equilibrated under an ethylene atmosphere, then treated with MAO (1.8 mL of a 10 % by weight solution in toluene) and stirred under 1 atm ethylene for 30 minutes. The reaction is quenched by the sequential addition of acetone, methanol, and 6M HCI. The precipitated polyethylene is isolated by filtration, washed, and dried *in vacuo.*

### Example 72

Polymerization of ethylene with catalyst system **IV**.

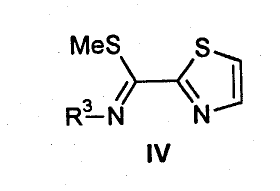

A 500 mL round bottom flask equipped with a magnetic stir bar, and a side-arm adapter with a Kontes high vacuum valve and a 24/40 septum, is flame-dried under vacuum (0.3 mm Hg), then (in the glovebox) charged with 10.0 mg of the ligand **IV,** 5.0 mg Ni(1,5-cyclooctadiene)₂ (Aldrich) and 106 mg B(C₆F₅)₃ (Strem). On the Schlenk line, the flask is evacuated and refilled with ethylene, then charged with 100 mL dry, deoxygenated toluene while being stirred at 21 C. After 30 min., the reaction is quenched by the addition of MeOH and acetone. The white flocculent polyethylene which separates is isolated by vacuum filtration and dried in vacuo (0.4 mm Hg, 6 h) to yield 5.0 g polyethylene.

The invention has been described above in detail with particular reference to preferred embodiments thereof, but it will be understood that variations and modifications other than as specifically described herein can be effected within the spirit and scope of the invention. Moreover, all patents and literature references or other references herein are hereby incorporated by reference.

## Claims

1. A catalyst comprising a compound of formula **I:** 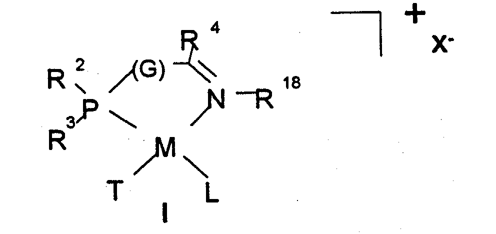 wherein:
R² and R³ are each independently selected from hydrocarbyl, or substituted hydrocarbyl, and in addition, R² and R³ may be taken together with phosphorus to form a ring;
G is hydrocarbyl or substituted hydrocarbyl;
R⁴ is hydrogen, hydrocarbyl, substituted hydrocarbyl, or silyl;
R¹⁸ is hydrocarbyl or substituted hydrocarbyl;
T is hydrogen or hydrocarbyl;
L is a monoolefin or a neutral Lewis base where the donating atom is nitrogen, oxygen, or sulfur;
M is Ni(II), Pd(II), or Co(II); and
X⁻ is a weakly coordinating anion.

2. A catalyst system wherein the catalyst is formed by contacting a first compound Y with a second compound **II** having the formula: 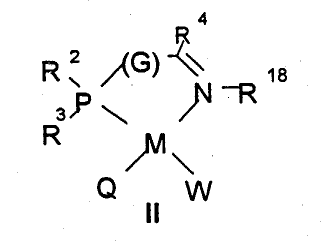 wherein:
R² and R³ are each independently selected from hydrocarbyl, or substituted hydrocarbyl, and in addition, R² and R³ may be taken together with phosphorus to form a ring;
G is hydrocarbyl or substituted hydrocarbyl;
R⁴ is hydrogen, hydrocarbyl, substituted hydrocarbyl, or silyl;
R¹⁸ is hydrocarbyl or substituted hydrocarbyl;
Q is hydrocarbyl, chloride, iodide or bromide;
W is hydrocarbyl, chloride, iodide or bromide;
M is Ni(II), Pd(II), or Co(II);
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

3. A compound of formula **III**: 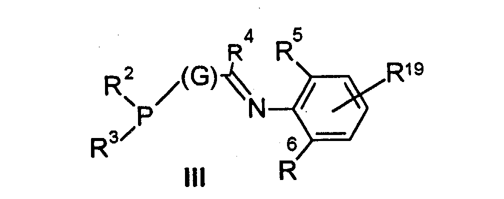 wherein
R² and R³ are each independently selected from hydrocarbyl, or substituted hydrocarbyl, and in addition, R² and R³ may be taken together with phosphorus to form a ring;
G is hydrocarbyl or substituted hydrocarbyl;
R¹⁹ is hydrogen, hydrocarbyl, substituted hydrocarbyl, heteroatom connected hydrocarbyl, or nitro;
R⁴ is hydrogen, hydrocarbyl, substituted hydrocarbyl, or silyl;
R⁵ and R⁶ are each independently selected from hydrogen, hydrocarbyl, substituted hydrocarbyl or halide; provided that R⁵ and R⁶ are not both hydrogen.

4. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst comprising a compound of formula **I:** 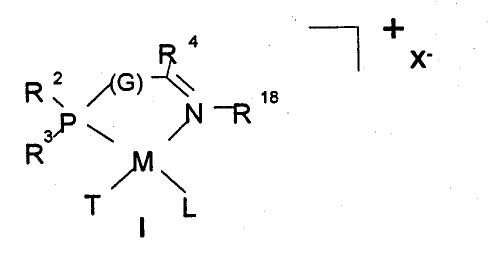 wherein:
R² and R³ are each independently selected from hydrocarbyl, or substituted hydrocarbyl, and in addition, R² and R³ may be taken together with phosphorus to form a ring;
G is hydrocarbyl or substituted hydrocarbyl;
R⁴ is hydrogen, hydrocarbyl, substituted hydrocarbyl, or silyl;
R¹⁸ is hydrocarbyl or substituted hydrocarbyl;
T is hydrogen or hydrocarbyl;
L is a monoolefin or a neutral Lewis base where the donating atom is nitrogen, oxygen, or sulfur;
M is Ni(II), Pd(II), or Co(II); and
X⁻ is a weakly coordinating anion;
at a temperature and a pressure sufficient to effect polymerization.

5. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst system wherein the catalyst is formed by contacting a first compound Y with a second compound II having the formula: 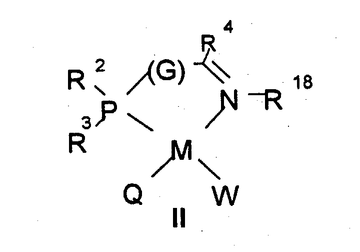 wherein:
R² and R³ are each independently selected from hydrocarbyl, or substituted hydrocarbyl, and in addition, R² and R³ may be taken together with phosphorus to form a ring;
G is hydrocarbyl or substituted hydrocarbyl;
R⁴ is hydrogen, hydrocarbyl, substituted hydrocarbyl, or silyl;
R¹⁸ is hydrocarbyl or substituted hydrocarbyl;
Q is hydrocarbyl, chloride, iodide or bromide;
W is hydrocarbyl, chloride, iodide or bromide;
M is Ni(II), Pd(II), or Co(II);
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

6. A process for the polymerization of olefins comprising contacting a compound of the formula **III** with a suitable nickel precursor, optionally a Lewis or Bronsted acid and one or more olefin monomers of the formula R'CH=CHR" wherein R' and R" are independently hydrogen, hydrocarbyl, fluoroalkyl, or R' and R" may be linked to form a cyclic olefin: 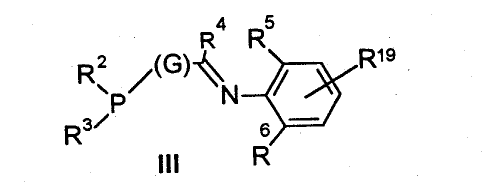 wherein
R² and R³ are each independently selected from hydrocarbyl, or substituted hydrocarbyl, and in addition, R² and R³ may be taken together with phosphorus to form a ring;
G is hydrocarbyl or substituted hydrocarbyl and may comprise a cyclic olefin;
R¹⁹ is hydrogen, hydrocarbyl, substituted hydrocarbyl, heteroatom connected hydrocarbyl, or nitro;
R⁴ is hydrogen, hydrocarbyl, substituted hydrocarbyl, or silyl; and
R⁵ and R⁶ are each independently selected from hydrogen, hydrocarbyl, substituted hydrocarbyl or halide; provided that R⁵ and R⁶ are not both hydrogen.

7. The process of claim 4 where the monomer of the formula R'CH=CHR" is ethylene.

8. The process of claim 5 where the monomer of the formula R'CH=CHR" is ethylene.

9. The process of claim 6 where the monomer of the formula R'CH=CHR" is ethylene.

10. A catalyst comprising a compound of formula **IV**: 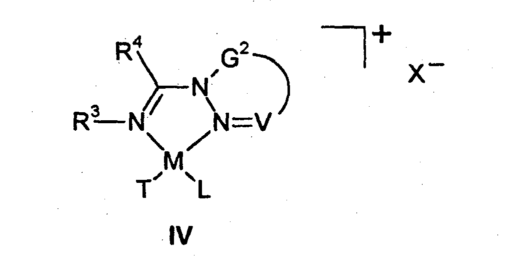 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
T is hydrogen, hydrocarbyl, or substituted hydrocarbyl;
L is a monoolefin or a neutral Lewis base where the donating atom is nitrogen, oxygen or sulfur;
M is Ni(II), Pd(II), or Co(II); and
X⁻ is a weakly coordinating anion.

11. A catalyst system wherein the catalyst is formed by contacting a first compound Y with a second compound V having the formula: 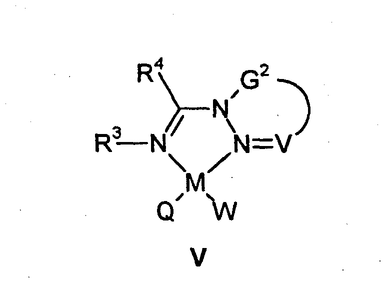 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
Q is hydrocarbyl, chloride, iodide, or bromide;
W is hydrocarbyl, chloride, iodide, or bromide;
M is Ni(II), Pd(II), or Co(II);
and Y is a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

12. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst comprising a compound of formula **IV:** 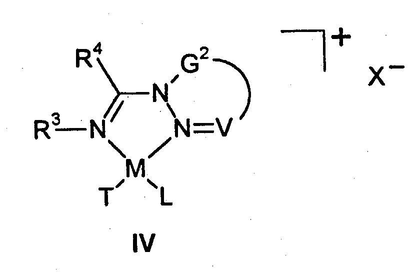 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
**V** is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
T is hydrogen, hydrocarbyl, or substituted hydrocarbyl;
L is a monoolefin or a neutral Lewis base where the donating atom is nitrogen, oxygen or sulfur;
M is Ni(II), Pd(II), or Co(II);
and X⁻ is a weakly coordinating anion.

13. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst wherein the catalyst is formed by contacting a first compound Y with a second compound V having the formula: 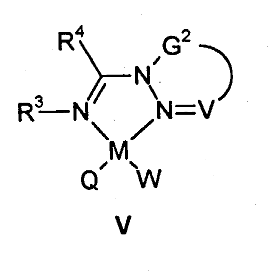 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
Q is hydrocarbyl, chloride, iodide, or bromide;
W is hydrocarbyl, chloride, iodide, or bromide;
M is Ni(II), Pd(II), or Co(II);
and Y is a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

14. A process for the polymerization of olefins comprising contacting a compound of the formula **VI** with a suitable nickel precursor, optionally a Lewis or Bronsted acid and one or more olefin monomers of the formula R'CH=CHR" wherein R' and R" are independently hydrogen, hydrocarbyl, fluoroalkyl, or R' and R" may be linked to form a cyclic olefin: 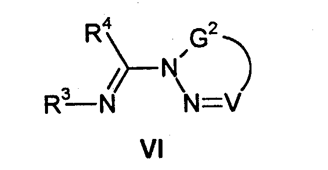 wherein:
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus.

15. The process of claim 12 where the monomer of the formula R'CH=CHR" is ethylene.

16. The process of claim 13 where the monomer of the formula R'CH=CHR" is ethylene.

17. The process of claim 14 where the monomer of the formula R'CH=CHR" is ethylene.

18. A catalyst comprising a compound of the formula **VII**: 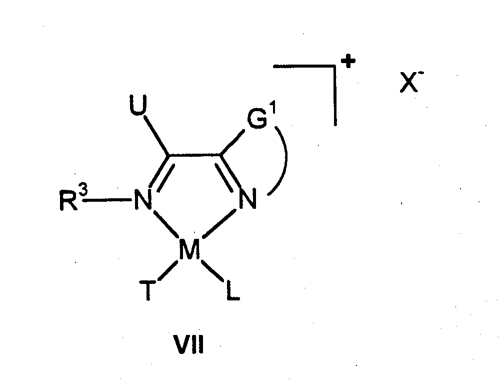 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N;
T is hydrogen, hydrocarbyl, or substituted hydrocarbyl;
L is a monoolefin or a neutral Lewis base where the donating atom is nitrogen, oxygen or sulfur;
M is Ni(II); Pd(II), or Co(II); and
X⁻ is a weakly coordinating anion.

19. A catalyst system wherein the catalyst is formed by contacting a first compound Y with a second compound VIII having the formula: 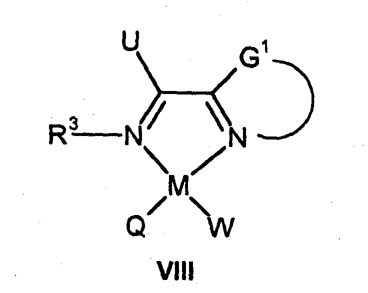 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N;
M is Ni(II), Pd(II); or Co(II);
Q is hydrocarbyl, chloride, iodide, or bromide;
W is hydrocarbyl, chloride, iodide, or bromide;
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

20. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst comprising a compound of the formula **VII**: 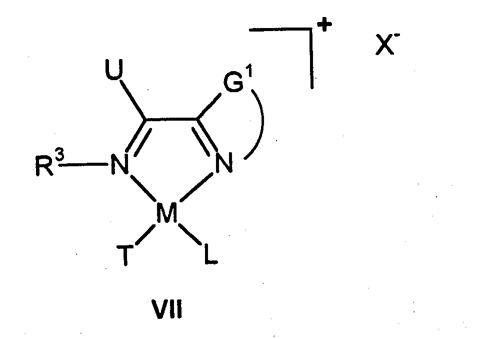 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N;
T is hydrogen, hydrocarbyl, or substituted hydrocarbyl;
L is a monoolefin or a neutral Lewis base where the donating atom is nitrogen, oxygen or sulfur;
M is Ni(II), Pd(II), or Co(II); and X⁻ is a weakly coordinating anion.

21. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst system wherein the catalyst is formed by contacting a first compound Y with a second compound VIII having the formula: 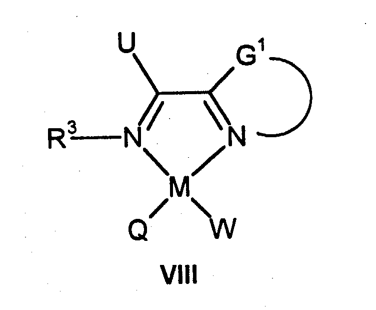 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N;
M is Ni(II), Pd(II), or Co(II);
Q is an hydrocarbyl, chloride, iodide, or bromide;
W is hydrocarbyl, chloride, iodide, or bromide;
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

22. The process as described in claim 21 where the Lewis acid is MAO.

23. A process for the polymerization of olefins comprising contacting a compound of the formula IX with a suitable nickel precursor, optionally a Lewis or Bronsted acid and one or more olefin monomers of the formula R'CH=CHR" wherein R' and R" are independently hydrogen, hydrocarbyl, fluoroalkyl, or R' and R" may be linked to form a cyclic olefin: 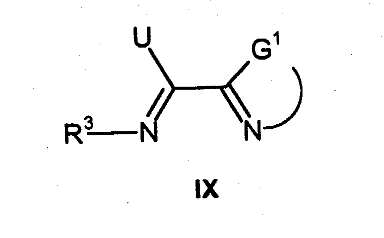 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N.

24. The process of claim 20 where the monomer is ethylene.

25. The process of claim 21 where the monomer is ethylene.

26. The process of claim 23 where the monomer is ethylene.

27. A catalyst comprising the formula **X**: 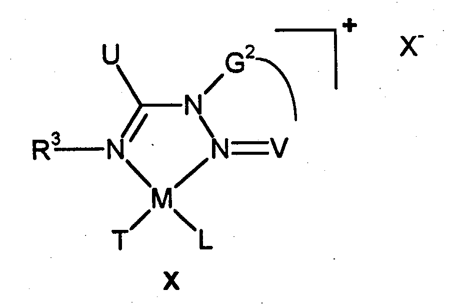 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N;
T is hydrogen, hydrocarbyl, or a substituted hydrocarbyl;
L is a monoolefin or a neutral Lewis base where the donating atom is nitrogen, oxygen, sulfur;
M is Ni(II), Pd(II), Co(II); and
X⁻ is a weakly coordinating anion.

28. A catalyst system wherein the catalyst is formed by contacting a first compound Y with a second compound **XI** having the formula: 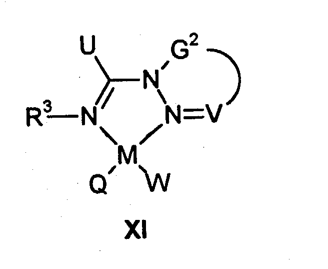 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N;
M is Ni(II), Pd(II); or Co(II);
Q is hydrocarbyl, chloride, iodide, or bromide;
W is hydrocarbyl, chloride, iodide, or bromide;
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

29. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst comprising the formula **X**: 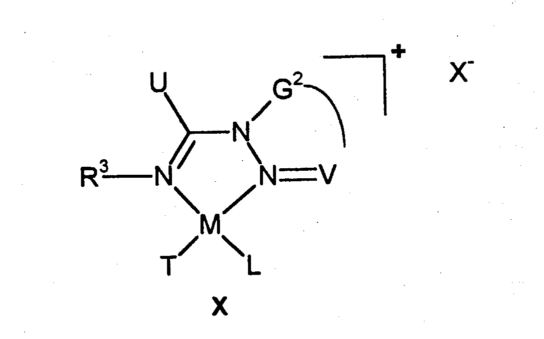 wherein:
R³ is hydrocarbyl, substituted hydrocarbyl or silyl;
U is OR⁴, SR⁴, SeR4 or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus:
G² is hydrocarbyl or substituted hydrocarbyl and may comprise a carbocyclic or heterocyclic ring, thereby forming a 5-membered or 6-membered heterocyclic ring comprising G², V, N, and N;
T is hydrogen, hydrocarbyl, or a substituted hydrocarbyl;
L is a monoolefin or a neutral Lewis base where the donating atom is nitrogen, oxygen, sulfur;
M is Ni(II), Pd(II), Co(II); and
X⁻ is a weakly coordinating anion.

30. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst wherein the catalyst is formed by contacting a first compound Y with a second compound **XI** having the formula: 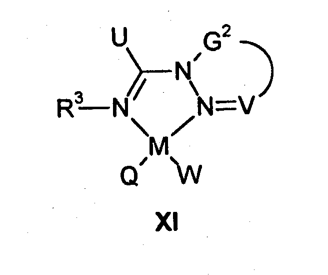 wherein:
R³ is hydrocarbyl, substituted hydrocarbyl or silyl;
U is OR⁴, SR⁴, SeR4 or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N;
M is Ni(II), Pd(II), or Co(II);
Q is hydrocarbyl, chloride, iodide, or bromide;
W is hydrocarbyl, chloride, iodide, or bromide;
and Y is selected from a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion.

31. The process as described in claim 30 where the Lewis acid is MAO.

32. A process for the polymerization of olefins comprising contacting a compound of the formula **XII** with a suitable nickel precursor, optionally a Lewis or Bronsted acid and one or more olefin monomers of the formula R'CH=CHR" wherein R' and R" are independently hydrogen, hydrocarbyl, fluoroalkyl, or R' and R" may be linked to form a cyclic olefin: 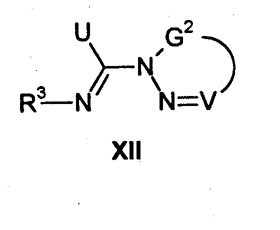 wherein:
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N.

33. The process of claim 29 where the monomer of the formula R'CH=CHR" is ethylene.

34. The process of claim 30 where the monomer of the formula R'CH=CHR"is ethylene.

35. The process of claim 32 where the monomer of the formula R'CH=CHR" is ethylene.

36. A catalyst comprising the formula XVI: 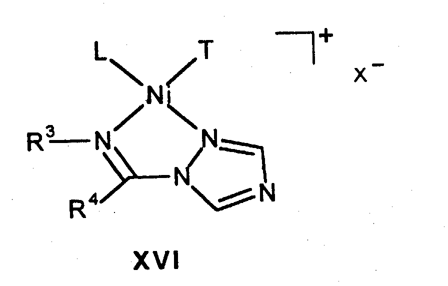 wherein:
R³ is hydrocarbyl or substituted hydrocarbyl;
R⁴ is hydrogen, hydrocarbyl, substituted hydrocarbyl, or silyl;
T is hydrogen, hydrocarbyl, or substituted hydrocarbyl;
L is a monoolefin or a neutral Lewis base where the donating atom is nitrogen, oxygen or sulfur; and
X⁻ is a weakly coordinating anion.

37. A process for preparing polyolefins comprising contacting one or more olefin monomers having the composition R'CH=CHR" wherein R' and R" are independently hydrogen, hydrocarbyl, fluoroalkyl, or R' and R" may be linked to form a cyclic olefin; with a catalyst comprising the formula **XVI**: 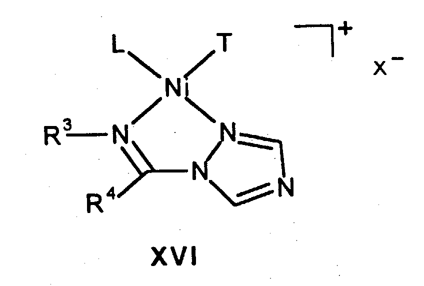 wherein:
R³ is hydrocarbyl or substituted hydrocarbyl;
R⁴ is hydrogen, hydrocarbyl, substituted hydrocarbyl, or silyl;
T is hydrogen, hydrocarbyl, or substituted hydrocarbyl;
L is a monoolefin or a neutral Lewis base where the donating atom is nitrogen, oxygen or sulfur; and
X⁻ is a weakly coordinating anion.

38. A catalyst system comprising a compound of the formula **XVII:** 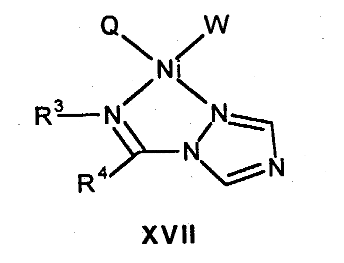 wherein:
R³ is hydrocarbyl or substituted hydrocarbyl;
R⁴ is hydrogen, hydrocarbyl, substituted hydrocarbyl, or silyl;
Q is hydrocarbyl, chloride, iodide, or bromide;
W is hydrocarbyl, chloride, iodide, or bromide.

39. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst system comprising a compound of the formula **XVII:** 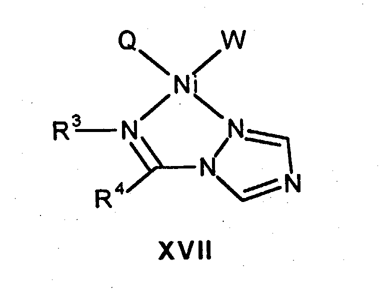 wherein:
R³ is hydrocarbyl or substituted hydrocarbyl;
R⁴ is hydrogen, hydrocarbyl, substituted hydrocarbyl, or silyl;
Q is hydrocarbyl, chloride, iodide, or bromide; and
W is hydrocarbyl, chloride, iodide, or bromide.

40. A process for the polymerization of olefins comprising contacting one or more monomers of the formula R'CH=CHR" with a binucleating or multinucleating ligand complexed to a Group 8-10 transition metal M and one or more Lewis acids, wherein the Lewis acid or acids are bound to one or more heteroatoms which are π-conjugated to the donor atom or atoms bound to the transition metal M; and R' and R" are each, independently selected from hydrogen, hydrocarbyl, fluoroalkyl, or may be linked to form a cyclic olefin.

41. The process of Claim 40 wherein the metal is nickel.

42. The process of Claim 41 wherein the Lewis acid is a boron or aluminum containing Lewis acid.

43. A composition comprising (i) a Group 8-10 transition metal M, (ii) one or more Lewis acids, and (iii) a binucleating or multinucleating compound of the formula **XVIII**: 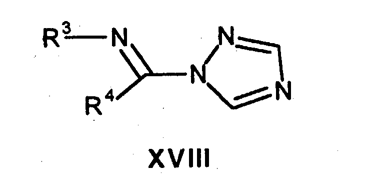 wherein the Lewis acid or acids are bound to one or more heteroatoms which are π-conjugated to the donor atom or atoms bound to the transition metal M;
R³ is hydrocarbyl or substituted hydrocarbyl;
R⁴ is hydrogen, hydrocarbyl, substituted hydrocarbyl, or silyl.

44. The composition of Claim 43 wherein the transition metal M is Ni(II), and the Lewis acid is a boron or aluminum containing Lewis acid.

45. A composition comprising (i) a Group 8-10 transition metal M, (ii) one or more Lewis acids, and (iii) a binucleating or multinucleating compound of the formula **VI**: 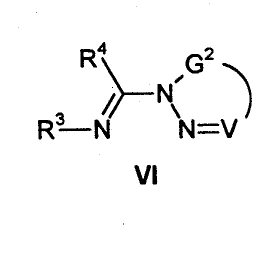 wherein the Lewis acid or acids are bound to one or more heteroatoms which are π-conjugated to the donor atom or atoms bound to the transition metal M;
R³ is hydrocarbyl or substituted hydrocarbyl;
R⁴ is hydrogen, hydrocarbyl, substituted hydrocarbyl, or silyl;
G² is hydrocarbyl or substituted hydrocarbyl and may comprise a carbocyclic or heterocyclic ring, thereby forming a 5-membered or 6-membered heterocyclic ring comprising G², V, N, and N;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus.

46. The composition of Claim 45 wherein the transition metal M is Ni(II), and the Lewis acid is a boron or aluminum containing Lewis acid.

47. A composition comprising (i) a Group 8-10 transition metal M, (ii) one or more Lewis acids, and (iii) a binucleating or multinucleating compound of the formula **IX**: 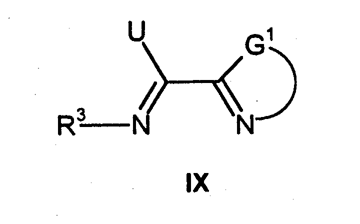 wherein the Lewis acid or acids are bound to one or more heteroatoms which are π-conjugated to the donor atom or atoms bound to a transition metal M;
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
G¹ is hydrocarbyl or substituted hydrocarbyl and may comprise a carbocyclic or heterocyclic ring, thereby forming a 5-membered or 6-membered heterocyclic ring comprising G¹, C, and N.

48. The composition of Claim 47 wherein the transition metal M is Ni(II), and the Lewis acid is a boron or aluminum containing Lewis acid.

49. A composition comprising (i) a Group 8-10 transition metal M, (ii) one or more Lewis acids, and (iii) a binucleating or multinucleating compound of the formula **XII**: 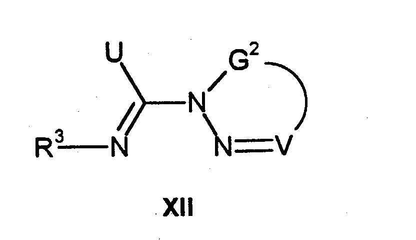 wherein the Lewis acid or acids are bound to one or more heteroatoms which are π-conjugated to the donor atom or atoms bound to a transition metal M;
R³ is hydrocarbyl, substituted hydrocarbyl or silyl;
U is OR⁴, SR⁴, SeR⁴ or NR⁴R⁸;
R⁴ and R⁸ are each independently selected from hydrogen, hydrocarbyl, substituted hydrocarbyl, silyl, and in addition, R⁴ and R⁸ may collectively form a ring with nitrogen;
V is CR⁶, N, or PR⁶R⁹;
R⁶ and R⁹ are each independently selected from hydrogen, hydrocarbyl, substituted hydrocarbyl, silyl or heteroatom connected hydrocarbyl, and in addition, R⁶ and R⁹ may collectively form a ring with phosphorus;
G² is hydrocarbyl or substituted hydrocarbyl and may comprise a carbocyclic or heterocyclic ring, thereby forming a 5-membered or 6-membered heterocyclic ring comprising G², V, N, and N.

50. The composition of Claim 49 wherein the transition metal M is Ni(II), and the Lewis acid is a boron or aluminum containing Lewis acid.

51. The catalyst of claim 1 or 2 where M is Ni(II).

52. The process of claim 4 or 5 where M is Ni(II).

53. The catalyst of claim 10 or 11 where M is Ni(II).

54. The process of claim 12 or 13 where M is Ni(II).

55. The process of claim 20 or 21 where M is Ni(II).

56. The process of claim 29 or 30 where M is Ni(II).

57. The catalyst of claim 18 or 19 where M is Ni(II).

58. The catalyst of claim 27 or 28 where M is Ni(II).
